# EUROPEAN PATENT SPECIFICATION

(11) **EP 1 608 786 B1**
(45) Date of publication and mention of the grant of the patent: **03.12.2008**
(21) Application number: 04758363.8
(22) Date of filing: 24.03.2004
(51) Int. Cl.: C12Q 1/68

(54) **GENOMIC PROFILING OF REGULATORY FACTOR BINDING SITES**
ERSTELLUNG EINES GENOMISCHEN PROFILS VON REGULATIONSFAKTOR-BINDUNGSSTELLEN
PROFILAGE GENOMIQUE DE SITES DE LIAISON DE FACTEURS REGULATEURS

(30) Priority: 28.03.2003 US 402689
(43) Date of publication of application: 28.12.2005
(73) Proprietor: Anesiva, Inc., South San Francisco, CA 94080 (US)
(72) Inventor: ZHANG, Jie, Campbell, CA 95008 (US); WEI, Hsiu-Ying, San Jose, CA 95120 (US); MCEVOY, Leslie, Margaret, Mountain View, CA 94043 (US)
(74) Representative: Forrest, Graham Robert
(86) International application number: PCT/US2004/009201
(87) International publication number: WO 2004/087966

(56) References cited:
- AERTS STEIN ET AL: "Toucan: Deciphering the cis-regulatory logic of coregulated genes." NUCLEIC ACIDS RESEARCH, vol. 31, no. 6, 15 March 2003 (2003-03-15), pages 1753-1764, XP002297446 ISSN: 0305-1048
- ZHU ZHOU ET AL: "Computational identification of transcription factor binding sites via a transcription-factor-centric clustering (TFCC) algorithm" JOURNAL OF MOLECULAR BIOLOGY, vol. 318, no. 1, 19 April 2002 (2002-04-19), pages 71-81, XP002297447 ISSN: 0022-2836
- LIANG J ET AL: "Computational analysis of microarray gene expression profiles: clustering, classification, and beyond" CHEMOMETRICS AND INTELLIGENT LABORATORY SYSTEMS, ELSEVIER SCIENCE PUBLISHERS B.V. AMSTERDAM, NL, vol. 62, no. 2, 28 May 2002 (2002-05-28), pages 199-216, XP004354187 ISSN: 0169-7439
- WASSERMAN W W ET AL: "Identification of regulatory regions which confer muscle-specific gene expression" JOURNAL OF MOLECULAR BIOLOGY, LONDON, GB, vol. 278, no. 1, 24 April 1998 (1998-04-24), pages 167-181, XP004453985 ISSN: 0022-2836
- BERMAN BENJAMIN P ET AL: "Exploiting transcription factor binding site clustering to identify cis-regulatory modules involved in pattern formation in the Drosophila genome" PROCEEDINGS OF THE NATIONAL ACADEMY OF SCIENCES OF THE UNITED STATES OF AMERICA, vol. 99, no. 2, 22 January 2002 (2002-01-22), pages 757-762, XP002297448 ISSN: 0027-8424
- ELKON RAN ET AL: "Genome-wide in silico identification of transcriptional regulators controlling the cell cycle in human cells." GENOME RESEARCH, vol. 13, no. 5, May 2003 (2003-05), pages 773-780, XP002297449 ISSN: 1088-9051

### SEQUENCE LISTING

<110> CORGENTECH, INC.
   ZHANG, Jie
   WEI, Hsiu-Ying
   MCEVOY, Leslie Margaret
<120> GENOMIC PROFILING OF REGULATORY FACTOR BINDING SITES
<130> 39753-0003 PCT
<140> Unassigned
   <141> Herewith
<150> US 10/402,689
   <151> 2003-03-28
<160> 65
<170> FastSEQ for windows version 4.0
<210> 1
   <211> 14
   <212> DNA
   <213> Homo sapiens
<220>
   <221> protein_bind
   <222> (1)...(14)
<400> 1
   aggggacttt ccca 14
<210> 2
   <211> 8 <212> DNA
   <213> Homo sapiens
<220>
   <221> protein_bind
   <222> (1)...(8)
<400> 2
   tttggcgg 8
<210> 3
   <211> 36
   <212> DNA
   <213> Homo sapiens
<220>
   <221> protein_bind
   <222> (10)...(22)
<400> 3
   agcgtcagaa ggggactttc ccaagagagg ccgaga 36
<210> 4
   <211> 21
   <212> DNA
   <213> Homo sapiens
<220>
   <221> protein_bind
   <222> (9)...(13)
<400> 4
   tgaacttgtc acgctttact g 21
<210> 5
   <211> 21
   <212> DNA
   <213> Homo sapiens
<220>
   <221> protein_bind
   <222> (12)...(16)
<400> 5
   aacttgtcac gctttactgt c 21
<210> 6
   <211> 21
   <212> DNA
   <213> Homo sapiens
<220>
   <221> protein_bind
   <222> (6)...(10)
<400> 6
   acttgtcacg ctttactgtc g 21
<210> 7
   <211> 21
   <212> DNA
   <213> Homo sapiens
<220>
   <221> protein_bind
   <222> (6)...(10)
<400> 7
   acttgtcacg ctttactgtc g 21
<210> 8
   <211> 9
   <212> DNA
   <213> Homo sapiens
<220>
   <221> protein_bind
   <222> (2)...(6)
<400> 8
   tcgataatg 9
<210> 9
   <211> 18
   <212> DNA
   <213> Homo sapiens
<220>
   <221> protein_bind
   <222> (11)...(15)
<400> 9
   cgataatgtg cattaagc 18
<210> 10
   <211> 18
   <212> DNA
   <213> Homo sapiens
<220>
   <221> protein_bind
   <222> (4)...(8)
<400> 10
   cgataatgtg cattaagc 18
<210> 11
   <211> 12
   <212> DNA
   <213> Homo sapiens
<220>
   <221> protein_bind
   <222> (4)...(8)
<400> 11
   gcattaagca aa 12
<210> 12
   <211> 14
   <212> DNA
   <213> Homo sapiens
<220>
   <221> protein_bind
   <222> (6)...(10)
<400> 12
   ctagttttat ttgt 14
<210> 13
   <211> 18
   <212> DNA
   <213> Homo sapiens
<220>
   <221> protein_bind
   <222> (6)...(10)
<400> 13
   agttttattt gtttattt 18
<210> 14
   <211> 15
   <212> DNA
   <213> Homo sapiens
<220>
   <221> protein_bind
   <222> (6)...(10)
<400> 14
   agttttattt gttta 15
<210> 15
   <211> 13
   <212> DNA
   <213> Homo sapiens
<220>
   <221> protein_bind
   <222> (6)...(10)
<400> 15 13
   gttttatttg ttt 13
<210> 16
   <211> 12
   <212> DNA
   <213> Homo sapiens
<220>
   <221> protein_bind
   <222> (3)...(7)
<400> 16 12
   ttttatttgt tt 12
<210> 17
   <211> 13
   <212> DNA
   <213> Homo sapiens
<220>
   <221> protein_bind
   <222> (4)...(8)
<400> 17 13
   ttttatttgt tta 13
<210> 18
   <211> 18
   <212> DNA
   <213> Homo sapiens
<220>
   <221> protein_bind
   <222> (6)...(10)
<400> 18
   ttatttgttt atttcatc 18
<210> 19
   <211> 15
   <212> DNA
   <213> Homo sapiens
<220>
   <221> protein_bind
   <222> (6)...(10)
<400> 19
   ttatttgttt atttc 15
<210> 20
   <211> 14
   <212> DNA
   <213> Homo sapiens
<220>
   <221> protein_bind
   <222> (7)...(11)
<400> 20
   tatttgttta tttc 14
<210> 21
   <211> 16
   <212> DNA
   <213> Homo sapiens
<220>
   <221> protein_bind
   <222> (7)...(11)
<400> 21
   tatttgttta tttcat 16
<210> 22
   <211> 11
   <212> DNA
   <213> Homo sapiens
<220>
   <221> protein_bind
   <222> (5)...(9)
<400> 22
   tatttgttta t 11
<210> 23
   <211> 11
   <212> DNA
   <213> Homo sapiens
<220>
   <221> protein_bind
   <222> (1)...(5)
<400> 23
   tatttgttta t 11
<210> 24
   <211> 14
   <212> DNA
   <213> Homo sapiens
<220>
   <221> protein_bind
   <222> (6)...(10)
<400> 24
   atttgtttat ttca 14
<210> 25
   <211> 13
   <212> DNA
   <213> Homo sapiens
<220>
   <221> protein_bind
   <222> (4)...(8)
<400> 25
   atttgtttat ttc 13
<210> 26
   <211> 13
   <212> DNA
   <213> Homo sapiens
<220>
   <221> protein_bind
   <222> (4)...(8)
<400> 26
   atttgtttat ttc 13
<210> 27
   <211> 12
   <212> DNA
   <213> Homo sapiens
<220>
   <221> protein_bind
   <222> (5)...(9)
<400> 27
   atttgtttat tt 12
<210> 28
   <211> 12
   <212> DNA
   <213> Homo sapiens
<220>
   <221> protein_bind
   <222> (3)...(7)
<400> 28
   atttgtttat tt 12
<210> 29
   <211> 11
   <212> DNA
   <213> Homo sapiens
<220>
   <221> protein_bind
   <222> (1)...(5)
<400> 29
   tgtttatttc a 11
<210> 30
   <211> 18
   <212> DNA
   <213> Homo sapiens
<220>
   <221> protein_bind
   <222> (12)...(16)
<400> 30
   gtttatttca tcttctaa 18
<210> 31
   <211> 12
   <212> DNA
   <213> Homo sapiens
<220>
   <221> protein_bind
   <222> (5)...(9)
<400> 31
   ttctaagtat aa 12
<210> 32
   <211> 24
   <212> DNA
   <213> Homo sapiens
<220>
   <221> protein_bind
   <222> (6)...(10)
<400> 32
   ttctaagtat aagaatacat tgta 24
<210> 33
   <211> 13
   <212> DNA
   <213> Homo sapiens
<220>
   <221> protein_bind
   <222> (5)...(9)
<400> 33
   agcattccca cca 13
<210> 34
   <211> 13
   <212> DNA
   <213> Homo sapiens
<220>
   <221> protein_bind
   <222> (5)...(9)
<400> 34
   agcattccca cca 13
<210> 35
   <211> 8
   <212> DNA
   <213> Homo sapiens
<220>
   <221> protein_bind
   <222> (2)...(6)
<400> 35
   gcgacaaa 8
<210> 36
   <211> 21
   <212> DNA
   <213> Homo sapiens
<220>
   <221> protein_bind
   <222> (12)...(16)
<400> 36
   agccctgcgc tccttacgac a 21
<210> 37
   <211> 10
   <212> DNA
   <213> Homo sapiens
<220>
   <221> protein_bind
   <222> (5)...(9)
<400> 37
   gcctcgtgcg 10
<210> 38
   <211> 12
   <212> DNA
   <213> Homo sapiens
<220>
   <221> protein_bind
   <222> (6)...(10)
<400> 38
   gcgggccaat cg 12
<210> 39
   <211> 21
   <212> DNA
   <213> Homo sapiens
<220>
   <221> protein_bind
   <222> (12)...(16)
<400> 39
   cgctgctccc gggtgatgac g 21
<210> 40
   <211> 21
   <212> DNA
   <213> Homo sapiens
<220>
   <221> protein_bind
   <222> (11) ... (15)
<400> 40
   tgctcccggg tgatgacgta g 21
<210> 41
   <211> 19
   <212> DNA
   <213> Homo sapiens
<220>
   <221> protein_bind
   <222> (5)...(9)
<400> 41
   gggtgatgac gtaggctgc 19
<210> 42
   <211> 12
   <212> DNA
   <213> Homo sapiens
<220>
   <221> protein_bind
   <222> (5)...(9)
<400> 42
   gtgatgacgt ag 12
<210> 43
   <211> 12
   <212> DNA
   <213> Homo sapiens
<220>
   <221> protein_bind
   <222> (3)...(7)
<400> 43
   gatgacgtag gc 12
<210> 44
   <211> 12
   <212> DNA
   <213> Homo sapiens
<220>
   <221> protein_bind
   <222> (3)...(7)
<400> 44
   gatgacgtag gc 12
<210> 45
   <211> 9
   <212> DNA
   <213> Homo sapiens
<220>
   <221> protein_bind
   <222> (5)...(9)
<400> 45
   tgacgtagg 9
<210> 46
   <211> 8
   <212> DNA
   <213> Homo sapiens
<220>
   <221> protein_bind
   <222> (1)...(5)
<400> 46
   tgacgtag 8
<210> 47
   <211> 8
   <212> DNA
   <213> Homo sapiens
<220>
   <221> protein_bind
   <222> (2)...(6)
<400> 47
   agggaggg 8
<210> 48
   <211> 8
   <212> DNA
   <213> Homo sapiens
<220>
   <221> protein_bind
   <222> (2)...(6)
<400> 48
   cttggcgg 8
<210> 49
   <211> 8
   <212> DNA
   <213> Homo sapiens
<220>
   <221> protein_bind
   <222> (3)...(7)
<400> 49 8
   cttggcgg 8
<210> 50
   <211> 8
   <212> DNA
   <213> Homo sapiens
<220>
   <221> protein_bind
   <222> (3)...(7)
<400> 50 8
   cttggcgg 8
<210> 51
   <211> 43
   <212> DNA
   <213> Homo sapiens
<400> 51
   tgaacttgtc acgctttact gtcgataatg tgcattaagc aaa 43
<210> 52
   <211> 48
   <212> DNA
   <213> Homo sapiens
<400> 52
   ctagttttat ttgtttattt catcttctaa gtataagaat acattgta 48
<210> 53
   <211> 13
   <212> DNA
   <213> Homo sapiens
<400> 53 13
   agcattccca cca 13
<210> 54
   <211> 28
   <212> DNA
   <213> Homo sapiens
<400> 54
   gcgacaaagc cctgcgctcc ttacgaca 28
<210> 55
   <211> 10
   <212> DNA
   <213> Homo sapiens
<400> 55
   gcctcgtgcg 10
<210> 56
   <211> 40
   <212> DNA
   <213> Homo sapiens
<400> 56
   gcgggcaatc gcgctgctcc cgggtgatga cgtaggctgc 40
<210> 57
   <211> 8
   <212> DNA
   <213> Homo sapiens
<400> 57
   agggaggg 8
<210> 58
   <211> 8
   <212> DNA
   <213> Homo sapiens
<400> 58
   cttggcgg 8
<210> 59
   <211> 2320
   <212> DNA
   <213> Homo sapiens
<400> 59
   gcgcagggag gggagacctt ggcggacggc ggagccccag cggaggtgaa agtattggcg 60
   gaaaggaaaa tacagcggaa aaatgcagag ctggagtcgt gtgtactgct ccttggccaa 120
   gagaggccat ttcaatcgaa tatctcatgg cctacaggga ctttctgcag tgcctctgag 180
   aacttacgca gatcagccga ttgatgctga tgtaacagtt ataggttctg gtcctggagg 240
   atatgttgct gctattaaag ctgcccagtt aggcttcaag acagtctgca ttgagaaaaa 300
   tgaaacactt ggtggaacat gcttgaatgt tggttgtatt ccttctaagg ctttattgaa 360
   caactctcat tattaccata tggcccatgg aacagatttt gcatctagag gaattgaaat 420
   gtccgaagtt cgcttgaatt tagacaagat gatggagcag aagagtactg cagtaaaagc 480
   tttaacaggt ggaattgccc acttattcaa acagaataag gttgttcatg tcaatggata 540
   tggaaagata actggcaaaa atcaagtcac tgctacgaaa gctgatggcg gcactcaggt 600
   tattgataca aagaacattc ttatagccac gggttcagaa gttactcctt ttcctggaat 660
   cacgatagat gaagatacaa tagtgtcatc tacaggtgct ttatctttaa aaaaagttcc 720
   agaaaagatg gttgttattg gtgcaggagt aataggtgta gaattgggtt cagtttggca 780
   aagacttggt gcagatgtga cagcagttga atttttaggt catgtaggtg gagttggaat 840
   tgatatggag atatctaaaa actttcaacg catccttcaa aaacaggggt ttaaatttaa 900
   attgaataca aaggttactg gtgctaccaa gaagtcagat ggaaaaattg atgtttctat 960
   tgaagctgct tctggtggta aagctgaagt tatcacttgt gatgtactct tggtttgcat 1020
   tggccgacga ccctttacta agaatttggg actagaagag ctgggaattg aactagatcc 1080
   tagaggtaga attccagtca ataccagatt tcaaactaaa attccaaata tctatgccat 1140
   tggtgatgta gttgctggtc caatgctggc tcacaaagca gaggatgaag gcattatctg 1200
   tgttgaagga atggctggtg gtgctgtgca cattgactac aattgtgtgc catcagtgat 1260
   ttacacacac cctgaagttg cttgggttgg caaatcagaa gagcagttga aagaagaggg 1320
   tattgagtac aaagttggga aattcccatt tgctgctaac agcagagcta agacaaatgc 1380
   tgacacagat ggcatggtga agatccttgg gcagaaatcg acagacagag tactgggagc 1440
   acatattctt ggaccaggtg ctggagaaat ggtaaatgaa gctgctcttg ctttggaata 1500
   tggagcatcc tgtgaagata tagctagagt ctgtcatgca catccgacct tatcagaagc 1560
   ttttagagaa gcaaatcttg ctgcgtcatt tggcaaatca atcaactttt gaattagaag 1620
   attatatatt tttttttctg aaatttcctg ggagcttttg tagaagtcac attcctgaac 1680
   aggatattct cacagctcca agaatttcta ggactgaatt atgaaacttt tggaaggtat 1740
   ttaataggtt tggacaaaat ggaatactct tatatctata ttttacataa atttagtatt 1800
   ttgtttcagt gcactaatat gtaagacaaa aaggactact tattgtagtc atcctggaat 1860
   atctccgtca actcatattt tcatgctgtt catgaaagat tcaatgcccc tgaatttaaa 1920
   tagctctttt ctctgataca gaaaagttga attttacatg gctggagcta gaatttgata 1980
   tgtgaacagt tgtgtttgaa gcacagtgat caagttattt ttaatttggt tttcacattg 2040
   gaaacaagtc agtcattcag atatgattca aatgtctata aaccaaactg atgtaagtaa 2100
   atggtctctc acttgtttta tttaacctct aaattctttc attttagggg tagcatttgt 2160
   gttgaagagg ttttaaagct tccattgttg tctgcaactc tgaagggtaa ttatatagtt 2220
   acccaaatta agagagtcta tttacggaac tcaaatacgt gggcattcaa atgtattaca 2280
   gtggggaatg aagatactga aataaacgtc ttaaatattc                       2320
<210> 60
   <211> 2108
   <212> DNA
   <213> Homo sapiens
<400> 60
   ggcacgaggg aggcgcccag cggaggtgaa agtattggcg gaaaggaaaa tacagcggaa 60
   aaatgcagag ctggagtcgt gtgtactgct ccttggccaa gagaggccat ttcaatcgaa 120
   tatctcatgg cctacaggga ctttctgcag tgcctctgag aacttacgca gatcagccga 180
   ttgatgctga tgtaacagtt ataggttctg gtcctggagg atatgttgct gctattaaag 240
   ctgcccagtt aggcttcaag acagtctgca ttgagaaaaa tgaaacactt ggtggaacat 300
   gcttgaatgt tggttgtatt ccttctaagg ctttattgaa caactctcat tattaccata 360
   tggcccatgg aaaagatttt gcatctagag gaattgaaat gtccgaagtt cgcttgaatt 420
   tagacaagat gatggagcag aagagtactg cagtaaaagc tttaacaggt ggaattgccc 480
   acttattcaa acagaataag gttgttcatg tcaatggata tggaaagata actggcaaaa 540
   atcaagtcac tgctacgaaa gctgatggcg gcactcaggt tattgataca aagaacattc 600
   ttatagccac gggttcagaa gttactcctt ttcctggaat cacgatagat gaagatacaa 660
   tagtgtcatc tacaggtgct ttatctttaa aaaaagttcc agaaaagatg gttgttattg 720
   gtgcaggagt aataggtgta gaattgggtt cagtttggca aagacttggt gcagatgtga 780
   cagcagttga atttttaggt catgtaggtg gagttggaat tgatatggag atatctaaaa 840
   actttcaacg catccttcaa aaacaggggt ttaaatttaa attgaataca aaggttactg 900
   gtgctaccaa gaagtcagat ggaaaaattg atgtttctat tgaagctgct tctggtggta 960
   aagctgaagt tatcacttgt gatgtactct tggtttgcat tggccgacga ccctttacta 1020
   agaatttggg actagaagag ctgggaattg aactagatcc cagaggtaga attccagtca 1080
   ataccagatt tcaaactaaa attccaaata tctatgccat tggtgatgta gttgctggtc 1140
   caatgctggc tcacaaagca gaggatgaag gcattatctg tgttgaagga atggctggtg 1200
   gtgctgtgca cattgactac aattgtgtgc catcagtgat ttacacacac cctgaagttg 1260
   cttgggttgg caaatcagaa gagcagttga aagaagaggg tattgagtac aaagttggga 1320
   aattcccatt tgctgctaac agcagagcta agacaaatgc tgacacagat ggcatggtga 1380
   agatccttgg gcagaaatcg acagacagag tactgggagc acatattctt ggaccaggtg 1440
   ctggagaaat ggtaaatgaa gctgctcttg ctttggaata tggagcatcc tgtgaagata 1500
   tagctagagt ctgtcatgca catccgacct tatcagaagc ttttagagaa gcaaatcttg 1560
   ctgcgtcatt tggcaaatca atcaactttt gaattagaag attatatata tttttttctg 1620
   aaatttcctg ggagcttttg tagaagtcac attcctgaac aggatattct cacagctcca 1680
   agaatttcta ggactgaatt atgaaacttt tggaaggtat ttaataggtt tggacaaaat 1740
   ggaatactct tatatctata ttttacataa atttagtatt ttgtttcagt gcactaatgt 1800
   gtaagacaaa aagctactta ttgtagcatc ctggaatatc tccgtcaact catattttca 1860
   tgctgttcat gaaagattca atgcccctga atttaaatag cttttttctc tgatacagaa 1920
   aagttgaatt ttacatggct ggagctagaa tttgatatgt gaacagttgt gtttgaagca 1980
   cagtgatcaa gttattttta atttggtttt cacattggaa acaagtcagt cattcagata 2040
   tgattcaaat gtctataaac cgaactgatg taagtaaaaa aaaaaaaaaa aaaaaaaaaa 2100
   aaaaaaaa 2108
<210> 61
   <211> 2341
   <212> DNA
   <213> Homo sapiens
<400> 61
   acgtaggctg cgcctgtgca tgcgcaggga ggggagacct tggcggacgg cggagcccca 60
   gcggaggtga aagtattggc ggaaaggaaa atacagcgga aaaatgcaga gctggagtcg 120
   tgtgtactgc tccttggcca agagaggcca tttcaatcga atatctcatg gcctacaggg 180
   actttctgca gtgcctctga gaacttacgc agatcagccg attgatgctg atgtaacagt 240
   tataggttct ggtcctggag gatatgttgc tgctattaaa gctgcccagt taggcttcaa 300
   gacagtctgc attgagaaaa atgaaacact tggtggaaca tgcttgaatg ttggttgtat 360
   tccttctaag gctttattga acaactctca ttattaccat atggcccatg gaacagattt 420
   tgcatctaga ggaattgaaa tgtccgaagt tcgcttgaat ttagacaaga tgatggagca 480
   gaagagtact gcagtaaaag ctttaacagg tggaattgcc cacttattca aacagaataa 540
   ggttgttcat gtcaatggat atggaaagat aactggcaaa aatcaagtca ctgctacgaa 600
   agctgatggc ggcactcagg ttattgatac aaagaacatt cttatagcca cgggttcaga 660
   agttactcct tttcctggaa tcacgataga tgaagataca atagtgtcat ctacaggtgc 720
   tttatcttta aaaaaagttc cagaaaagat ggttgttatt ggtgcaggag taataggtgt 780
   agaattgggt tcagtttggc aaagacttgg tgcagatgtg acagcagttg aatttttagg 840
   tcatgtaggt ggagttggaa ttgatatgga gatatctaaa aactttcaac gcatccttca 900
   aaaacagggg tttaaattta aattgaatac aaaggttact ggtgctacca agaagtcaga 960
   tggaaaaatt gatgtttcta ttgaagctgc ttctggtggt aaagctgaag ttatcacttg 1020
   tgatgtactc ttggtttgca ttggccgacg accctttact aagaatttgg gactagaaga 1080
   gctgggaatt gaactagatc ctagaggtag aattccagtc aataccagat ttcaaactaa 1140
   aattccaaat atctatgcca ttggtgatgt agttgctggt ccaatgctgg ctcacaaagc 1200
   agaggatgaa ggcattatct gtgttgaagg aatggctggt ggtgctgtgc acattgacta 1260
   caattgtgtg ccatcagtga tttacacaca ccctgaagtt gcttgggttg gcaaatcaga 1320
   agagcagttg aaagaagagg gtattgagta caaagttggg aaattcccat ttgctgctaa 1380
   cagcagagct aagacaaatg ctgacacaga tggcatggtg aagatccttg ggcagaaatc 1440
   gacagacaga gtactgggag cacatattct tggaccaggt gctggagaaa tggtaaatga 1500
   agctgctctt gctttggaat atggagcatc ctgtgaagat atagctagag tctgtcatgc 1560
   acatccgacc ttatcagaag cttttagaga agcaaatctt gctgcgtcat ttggcaaatc 1620
   aatcaacttt tgaattagaa gattatatat ttttttttct gaaatttcct gggagctttt 1680
   gtagaagtca cattcctgaa caggatattc tcacagctcc aagaatttct aggactgaat 1740
   tatgaaactt ttggaaggta tttaataggt ttggacaaaa tggaatactc ttatatctat 1800
   attttacata aatttagtat tttgtttcag tgcactaata tgtaagacaa aaaggactac 1860
   ttattgtagt catcctggaa tatctccgtc aactcatatt ttcatgctgt tcatgaaaga 1920
   ttcaatgccc ctgaatttaa atagctcttt tctctgatac agaaaagttg aattttacat 1980
   ggctggagct agaatttgat atgtgaacag ttgtgtttga agcacagtga tcaagttatt 2040
   tttaatttgg ttttcacatt ggaaacaagt cagtcattca gatatgattc aaatgtctat 2100
   aaaccaaact gatgtaagta aatggtctct cacttgtttt atttaacctc taaattcttt 2160
   cattttaggg gtagcatttg tgttgaagag gttttaaagc ttccattgtt gtctgcaact 2220
   ctgaagggta attatatagt tacccaaatt aagagagtct atttacggaa ctcaaatacg 2280
   tgggcattca aatgtattac agtggggaat gaagatactg aaataaacgt cttaaatatt 2340
   c                                                                  2341
<210> 62
   <211> 301
   <212> DNA
   <213> Homo sapiens
<400> 62
   gttgaacttg tcacgcttta ctgtcgataa tgtgcattaa gcaaacgcta gttttatttg 60
   tttatttcat cttctaagta taagaataca ttgtagctcg acattttggc accagcccct 120
   aaagcattcc caccaccacc cccgctgcga caaagccctg cgctccttac gacagcgtac 180
   gacgccgagc ctgacaggaa cgcctcgtgc ggtagaaccg cgcgggccaa tcgcgctgct 240
   cccgggtgat gacgtaggct gcgcctgtgc atgcgcaggg aggggagacc ttggcggagc 300
   g                                                                  301
<210> 63
   <211> 21
   <212> DNA
   <213> Homo sapiens
<220>
   <221> protein_bind
   <222> (9)...(13)
<400> 63
   tgaacttatc acgctttact g 21
<210> 64
   <211> 18
   <212> DNA
   <213> Homo sapiens
<220>
   <221> protein_bind
   <222> (4)...(8)
<400> 64
   caataatgtg cattaagc 18
<210> 65
   <211> 12
   <212> DNA
   <213> Homo sapiens
<220>
   <221> protein_bind
   <222> (5)...(8)
<400> 65
   agcattccac ca 12

## Description

### BACKGROUND OF THE INVENTION

### Field of the Invention

The present invention relates generally to methods that provide profiles of regulatory factor binding sites of all the known genes, and more particularly to methods for identifying and characterizing regulatory factors binding sites in order to develop systematical analysis on identified binding sites for further therapeutic strategies development.

### Description of the Related Art

Altering gene expression level has become an important and efficient approach to address the human disorders. The expression level of each gene is controlled by the transcription machinery, in which some specific proteins called transcription factors (TFs) bind to the regulatory region of the gene, and in turn to initialize the transcription processes. Thus, the corresponding TFs and their binding sites on gene regulatory region can play the essential role in controlling the transcription level of gene. Therefore, transcription factors and their related transcription mechanisms have become the "hot" spots in modem bio-medical research and development efforts.

For each gene, the transcription starting site (TSS) is the position where its' mRNA starts to be transcribed from DNA by RNA polymerase II. During this process, the gene regulatory region is associated and bound by certain regulatory factors. These bound factors together with other transcription proteins formed a transcription complex that can initialize the transcription process. More specifically, this typically includes the transcription factor binding sites that are the short consensus genomic sequences. One of the most important regulatory regions is the core promoter usually located immediately before or flanking TSS. Thus, identifying TSS is important to define the transcription regulatory region for each gene. Currently, many specific researches and developments focus their efforts on the specific TFs and corresponding binding sites, which provided many solid data but still failed to meet the large requirement of the development of genomic-related biomedical needs. To meet the fast growing transcription factors related drug discovery business and challenge, it is very important to identify all putative regulatory factors and characterize their corresponding binding sites in genome. Especially, with the finish of human genome project and occurrences of large amount of disease-related gene expression data (such as microarray-based data), the genome wide profiling of regulatory factor binding sites become urgent

The present invention retrieved all the full-length genes from various public available databases (such as, NCBI refseq, NIH MGC consortium, DBTSS database of Japan, and so on) and then mapped these gene's TSSs on the most updated Human Genome Working Draft (such as Assembly version July, 2003, or NCBI build 34). Then it defined the most upstream TSS for each gene by comparing all the possible TSSs generated by mapping the position of this gene. The transcription regulatory region (TRR), such as core promoter regions were defined based on the most 5' TSS positions, and their corresponding genomic sequences were retrieved from most updated human genome for further analysis. The profiled TRR for all the known genes were stored in a database for further drug-target related statistic analysis and for further therapeutic strategies development.

### SUMMARY OF THE INVENTION

Accordingly, an object of the present invention is to provide improved methods for genomic-profiling regulatory factor binding sites.

In another object of the present invention, methods for profiling regulatory factor binding sites, are provided that employ genome-wide probability mapping relative to profiled binding sites.

Yet another object of the present invention is to provide improved methods for biomedical research.

A further object of the present invention is to provide improved methods for preclinical development.

Still another object of the present invention is to provide improved methods for drug screening applications.

Another object of the present invention is to provide improved methods for target discovering and target validation.

Yet another object of the present invention is to provide improved methods for profiling of a regulatory region.

A further object of the present invention is to provide improved methods for building the genome or tissue wide connections between regulatory profilings of different genes.

Still a further object of the present invention is to provide improved methods for understanding the genome or tissue or cell background of various known transcription profiling. In first aspect, the present invention relates to a method for profiling regulatory factor binding sites, comprising
locating and retrieving a complete and most 5' full-length gene for mapping the regulatory regions of said gene to a human genome;
retrieving genomic sequences from the human genome for the gene regulatory region comprising the sequence about 200-300 bases upstream of the transcriptional start site (TSS) and the sequence about 50-100 bases downstream of the TSS;
screening DNA sequence information of the retrieved gene regulatory region for the purpose of identifying putative regulatory factor binding sites;
creating a conservation score for each putative regulatory factor binding site;
profiling the putative regulatory factor binding sites of the gene;
collecting profiles including transcription factor binding site sequence information and the conservation score in a database; and
computationally generating all the possible transcriptional decoys for profiled regulatory factor binding sites.

### BRIEF DESCRIPTION OF THE DRAWINGS

Figure 1 is a flow chart illustrating a method for profiling regulatory factor binding sites.
Figure 2 is a flow chart that described how to define the transcription regulatory region of a gene (example Gene X).
Figure 3 is a flow chart illustrating calculating the frequency of TF binding sites.
Figure 4 illustrates that the core promoter region can include 200-300 bases upstream and about 50-100 bases downstream of the TSS.
Figure 5 is a description of one embodiment of a structure of a database.
Figure 6 is a flow chart illustrating the Figure 5 database.
Figure 7 lists the complete sequences for gene DLD retrieved from the refseq database (SEQ ID NO 59).
Figure 8 lists the complete sequences for gene DLD retrieved from the MGC database (SEQ ID NO 60).
Figure 9 lists the complete sequences for gene DLD retrieved from the DBTSS database (SEQ ID NO 61).
Figure 10 lists the stored sequence for gene DLD (SEQ ID NO 62).
Figure 11 is a screen shot of a query form that can be used with the Figure 7 database.
Figure 12 is a screen shot of one embodiment of a database query result from the Figure 5 database.
Figure 13 illustrates one embodiment of a system for displaying profiled regulatory factor binding sites.

### DESCRIPTION OF THE PREFERRED EMBODIMENTS

In various embodiments, the present invention provides methods for genome wide profiling regulatory factor binder sites. Examples of regulatory factor binder sites include but are not limited to, sequence AGGGGACTTTCCCA (SEQ ID NO 1) as the binding sites for transcription factor NF-kappa B; sequence TTTGGCGG (SEQ ID NO 2) as the binding sites for transcription factor E2F-1, and the like.

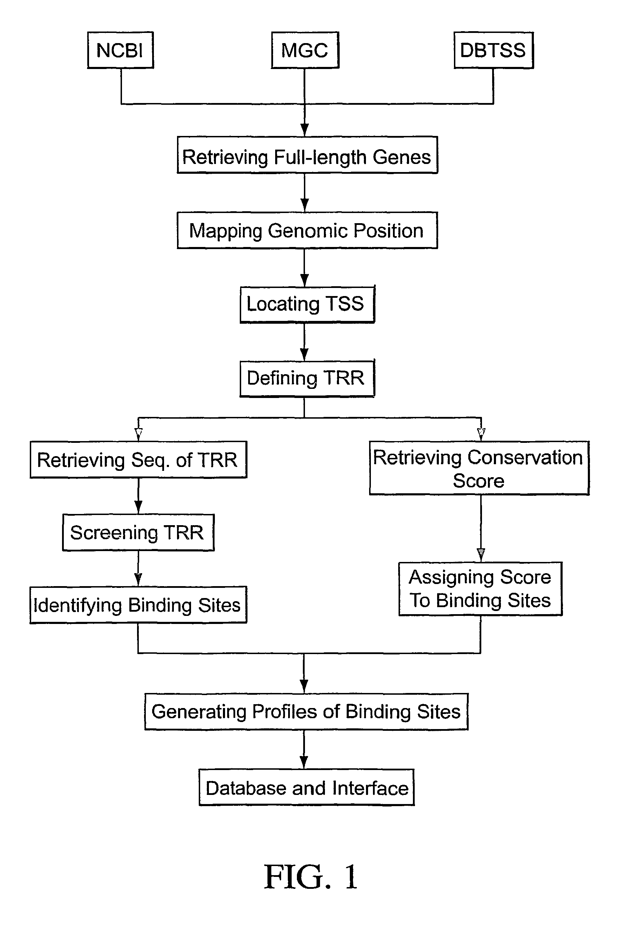

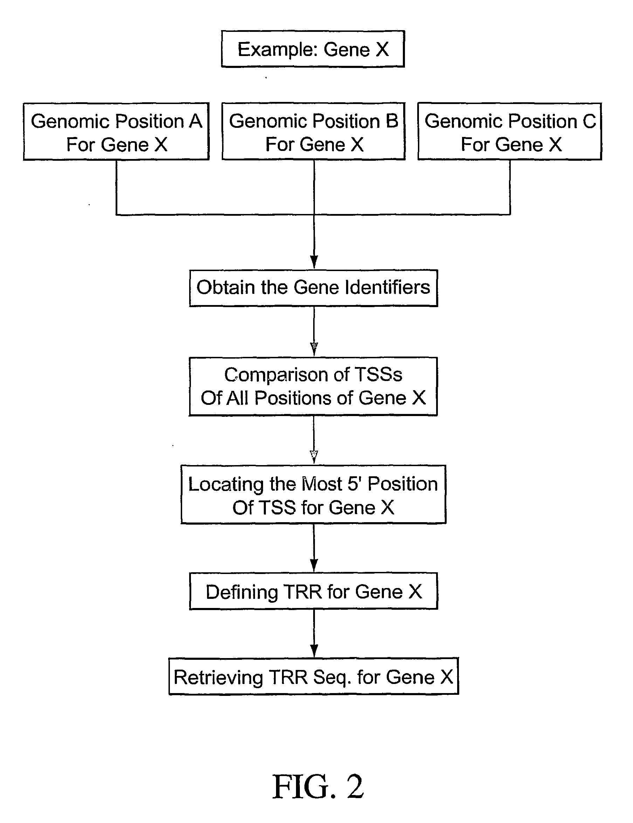

Referring to the flow charts of Figures 1 and 2, in one embodiment of the present invention, genomic sequences of gene regulatory regions are retrieved and are mapped to human genome. Based on the mapped genes, the most 5 prime position of TSS for each gene is identified and the corresponding regulatory region for the gene is identified. DNA sequence information for each retrieved gene regulatory region is screened to identify putative regulatory factor binding sites. The putative regulatory factor binding sites are then profiled.

Information retrieved from the database can be utilized for a variety of different purposes and applications including but not limited to, biomedical research, pre-clinical development, drug screening applications, target discovering and target validation, profiling of a regulatory region, building the genome or tissue wide connections between regulatory profilings of different genes, understanding the genome or tissue background of various known transcription profiling understanding the genome or tissue background of various known transcription profiling, and the like.

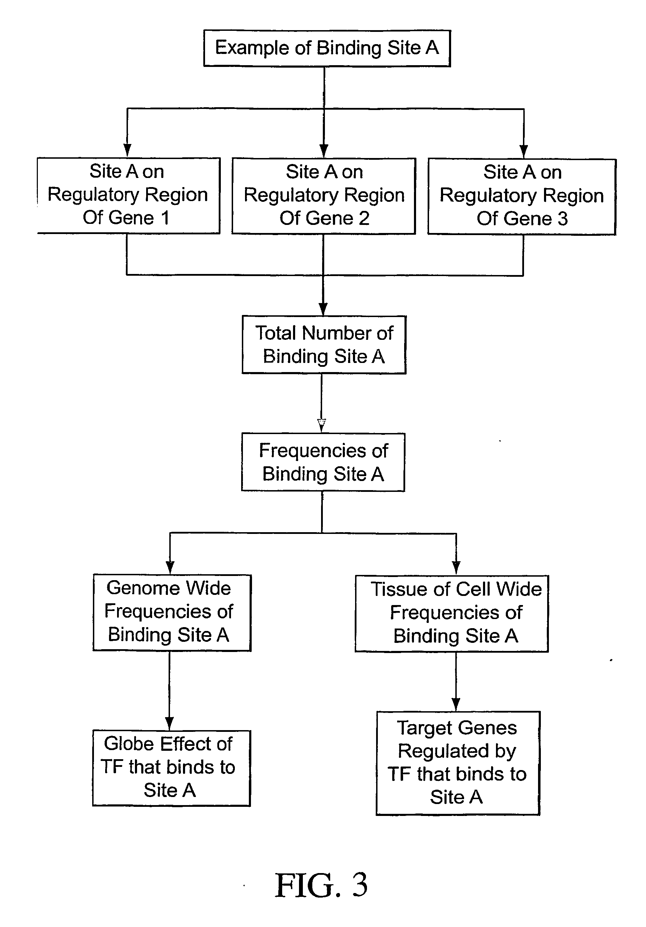

Referring to Figure 3, probability mapping is applied to the identified binding sites. The probability mapping describes the identification of existences of a specific transcription regulatory factor binding sites, such as all the putative E2F-1 sites, in the regulatory region of all the genes or in the genes that expressed in certain tissue or cell. The probability mapping tells how many genes are possibly transcription-regulated by a specific regulatory factor. It also indicates that how much biological system wide, genome wide, cell wide, or tissue wide, effect a specific regulator factor could have. This information is very useful for bio-medical research based therapeutic method development.

In another embodiment of the present invention a full-length gene is mapped for purposes of mapping gene regulatory regions. It will be appreciated that for purposes of this specification, full length extends to the length of the gene. This can cause a slight shift of the genomic position of the transcription start sites of the different versions of the same gene. In one embodiment, all of the available full-length gene is used in a comparison in order to obtain the most 5' TSS. Based on the most 5' TSS, the regulatory regions of genes are defined and the genomic sequences of gene regulatory regions are retrieved. DNA sequence information is screened for each retrieved gene regulatory region to identify putative regulatory factor binding sites. The putative regulatory factor binding sites are mapped to the human genome.

Full-length genes are retrieved to provide sequences information for retrieved genes. The retrieved genes can be mapped to a recently updated human genome using a tool provided by a public available UCSC genome browser databases, self-developed scripts, and the like. In one embodiment, the transcription start site is mapped. In one embodiment, the TSS is mapped by taking the most 5' TSS of each gene after comparing all available TSS's for the gene, illustrated in Figure 2.

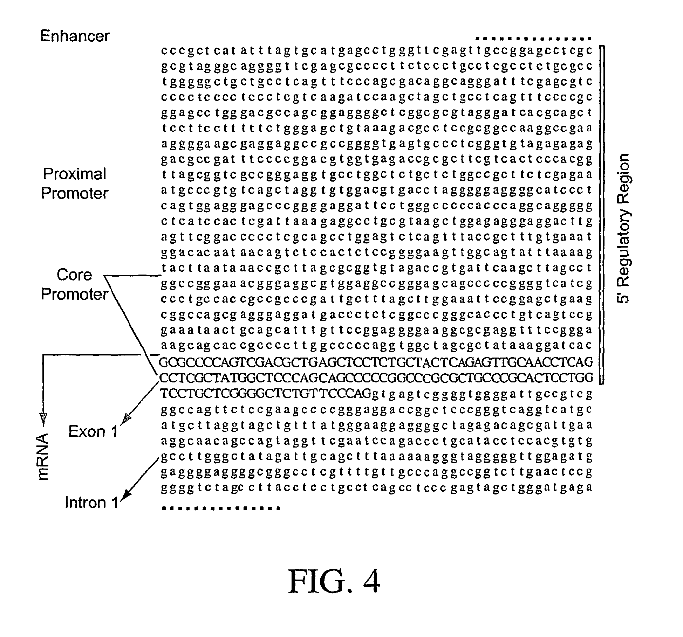

A genomic sequence of a regulatory region can be retrieved for each retrieved gene with the most 5' TSS from the most updated human genome. The 5' regulatory region is the sequences upstream of the TSS and downstream of the TSS. In various embodiments, the gene regulatory regions include but are not limit to, the core promoter region, the upstream enhancer region, a downstream regulatory region, and the like, as illustrated in Figure 4. Methods of the invention retrieve genomic sequences comprising a core promoter region including 200-300 bases upstream and about 50-100 bases downstream of the TSS.

Corresponding sequences relative to TSS can be cut and stored. The corresponding sequences relative to TSS can be cut and stored with the use of self-developed scripts from genomic sequences based on a specific release, older, updated and future releases, including but not limited to the UCSC genome browser, NCBI genome database, the Ensembl database, other genomic sequence databases and the like.

In one embodiment, the DNA sequence information is screened using a MATCH program that is licensed from TRANSFAC database. The DNA sequence information screening can include selecting the TF matrix, scores of matrix similarity, scores of core similarity, and the like.

Cut-off may be applied to reduce the false positive and false negative matching during screening. A genomic or tissue-specific frequency of each binding site and can be determined. The frequency can be the existence of specific TF binding sites in regulatory regions of at least one of, (i) all the genes genome wide, (ii) all the genes specific cell wide, (iii) all the genes specific-tissue wide, (iv) all the genes specific-defined. The frequency can be the existence of specific TF binding sites in regulatory regions of tissue specific genes. Additionally, the frequency can also be considered with a conservation score or an expression level score. By way of illustration, and without limitation, the identified binding sites can be considered differently based on their corresponding conservation score or their corresponding gene expression level. For example, a binding site with higher conservation score or the corresponding gene with higher expression level could play a more significant role than those with lower scores.

In methods of the invention, The conservation score for each binding site is created. The conservation score is selected to cover regions where the TF binding sites are identified as well as any other measurements that indicate conservation levels between the two species including but not limited to mouse and human. The position of each binding site can be determined. The position can be based on a human genome working draft. The position is a converted position in a human genome working draft. As more sequence pieces are added, the total length for each chromosome grows. This shifts the position reading for each base on the chromosome. However, the position can be easily converted and the relative position of a regulatory region to the position of the gene remains unchanged. The genome position of a start and end can be determined. A distance of each binding site to the TSS can be determined. The distance is relative to a number of bases between a binding site and the TSS. By way of illustration, and without limitation, in one embodiment the distance is that of the last base between defined binding sites to the base of TSS's 23 base. In this example, there are 23 bases between these two specific bases.

In one embodiment of the present invention, based on the positions of most 5 prime TSSs, the 5 prime regulatory sequences from most updated Human Genome Working Draft are retrieved for all the available genes using self developed computer scripts and programs. These retrieved sequences include but not limited to 250-base 5 prime upstream and 50-base 3 prime downstream of TSS for each gene.

All the regulatory region sequences can be analyzed using well-characterized transcription factor binding consensus sequence patterns (or, position weighted matrix) created by licensed TRANSFAC databases (TRANSFAC professional 6.3 version, Wingender et al., Nucleic Acids Res. 29,281). The sites with high score matching with binding matrix will be selected. These sites include their positions in the genome (relative to specific genome assemble version) and their lengths and their synergism information with flanking sites.

All the binding sites result from above are further analyzed by comparing their conservation scores with mouse. The mouse genome and relative conservation information will be retrieved from public available NCBI and UCSC genome databases, and the conservation comparison with human transcription factor binding sites will be done using self-generated scripts and programs.

The resulted transcription factor binding site sequences information from above, include their genomic positions (start, end), length, distant to TSS of each gene, and the flanking regions (include but not limit to 10-base both 5 prime and 3 prime) will be deposited into a database. The related reference links such as gene name, function, annotation, et al are also added.

In methods of the present invention, All the possible transcription decoys are computational generated based on the database. The decoys can further be experimentally screened by using high-throughput methods, such as oligo-array, capillary-electrophoresis, et al for binding efficiency optimization. All the optimized decoy information will be deposited into the database. The partial information in the database can be used in future versions of the database.

Profiles of the regulatory regions of genes include but are not limited to, (i) probability mapping of each regulatory factor binding site, (ii) target genes identification for each known regulatory factor; (iii) statistic analysis of regulatory factor binding profiles of genes identified from various differential expressed genes, and the like.

In one embodiment, a length of each binding site is determined. Sequence information about regions adjacent to the binding site can also be determined. Again by illustration and without limitation, one example is agcgtcagaAGGGGACTTTCCCaagagaggccgaga, (SEQ ID NO 3) with the small case base letters flanking the core binding sites, in upper case.

Co-existence information of other binding sites can also be ascertained. The transcription machinery usually requires the formation of the complex by several different transcription related proteins and includes the several different DNA binding factors. When the present invention, the binding sites are profiled for a regulatory region of the gene and often more than one binding site is identified from a single region. The number of binding sites can be, by way of example, fifteen to twenty from a single region. The cluster of the binding sites and their positions can be determined.

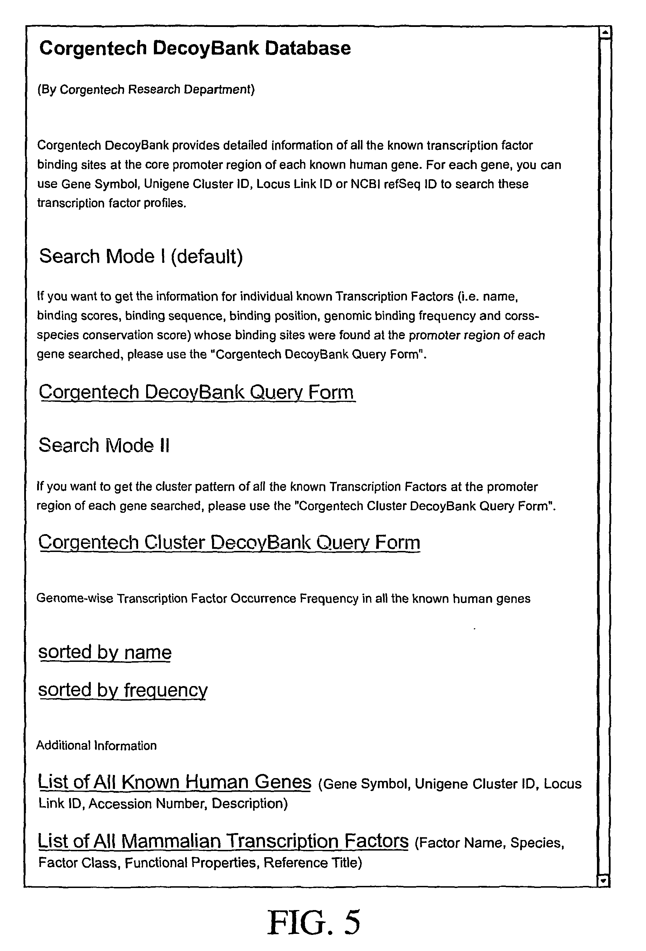

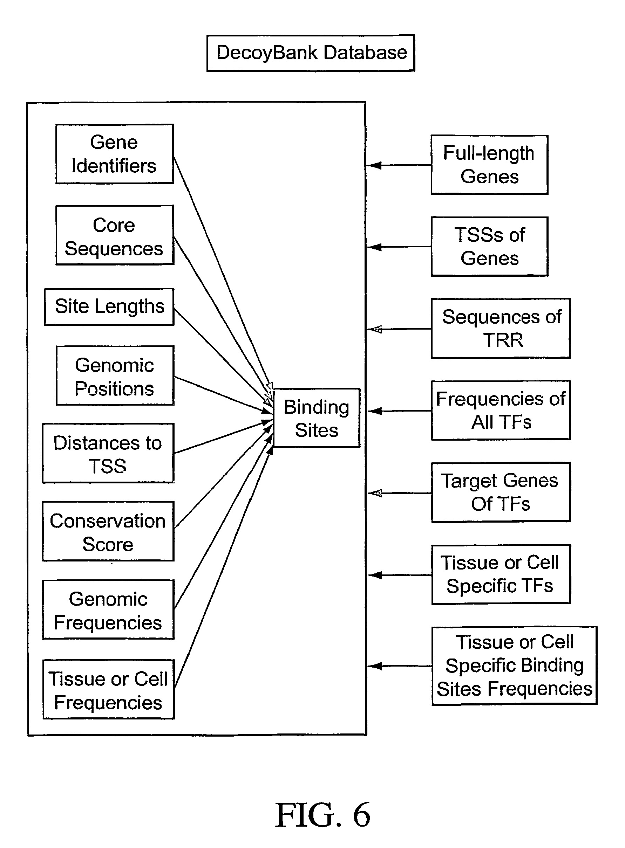

Referring now to Figures 5 and 6, the present application also describes a data structure tangibly stored on a computer readable medium that includes a database with the profiled identified binding site information. The database includes a core table with identifiers, binding sites and the like. Binding site information includes but is not limited to, sequence, length, position, direction, frequency, and the like. One supporting table includes TSS position of all genes. A sequence table provides the sequences of regulatory regions of genes. Additional support tables include but are not limited to frequency of TF, target genes of TF for each TF, and the like.

All of the tables are linked by one or more identifiers. In one embodiment, several instead of one perl CGI script are used to reach and search the database and then display the corresponding information. A web-browser interface is provided.

The database is searchable by a variety of different means, including but not limited to gene identifiers, gene symbol, or self-developed identifiers and the like. Gene identifiers can be selected from the NCBI database, which can be a, Unigene Cluster ID, LoucsLink ID, international approved gene symbols, and the like.

In oneexample, the database includes genomic frequencies information for TF, and can be sorted by at least a TF name or TF frequencies. The TF frequencies can include genome frequencies and tissue specific frequencies. In one specific example, the database contains the profiles of regulatory factor binding sites for all the known genes (about 15,450 total).

By way of illustration, and without limitation, one gene (symbol: DLD, dihydrolipoamide dehydrogenase) is used to briefly show how the database is built.

### 1. Retrieving of full length genes for an example gene DLD to provide sequences information.

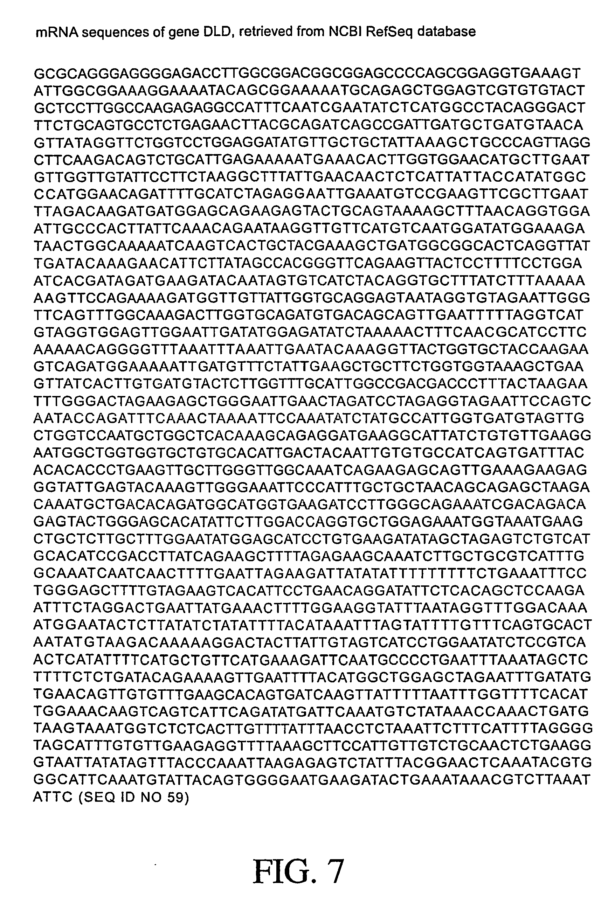

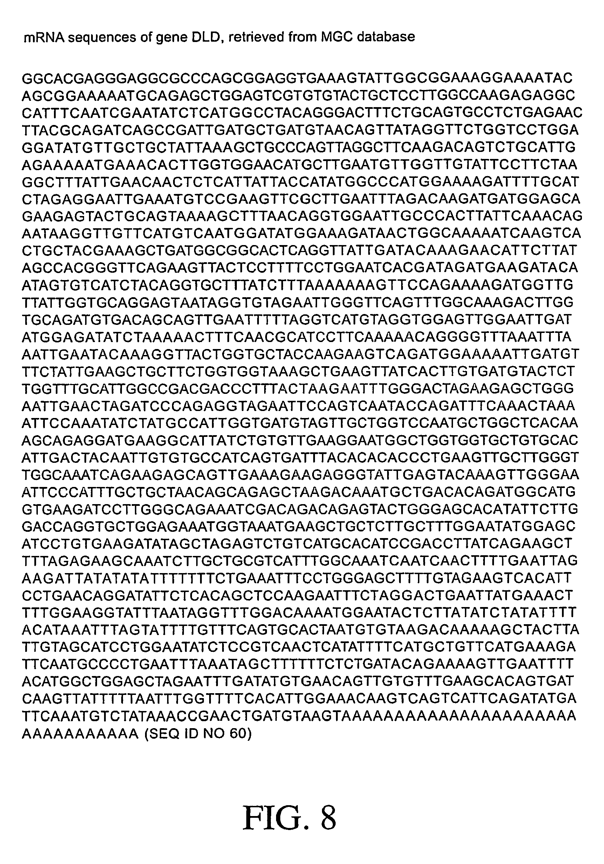

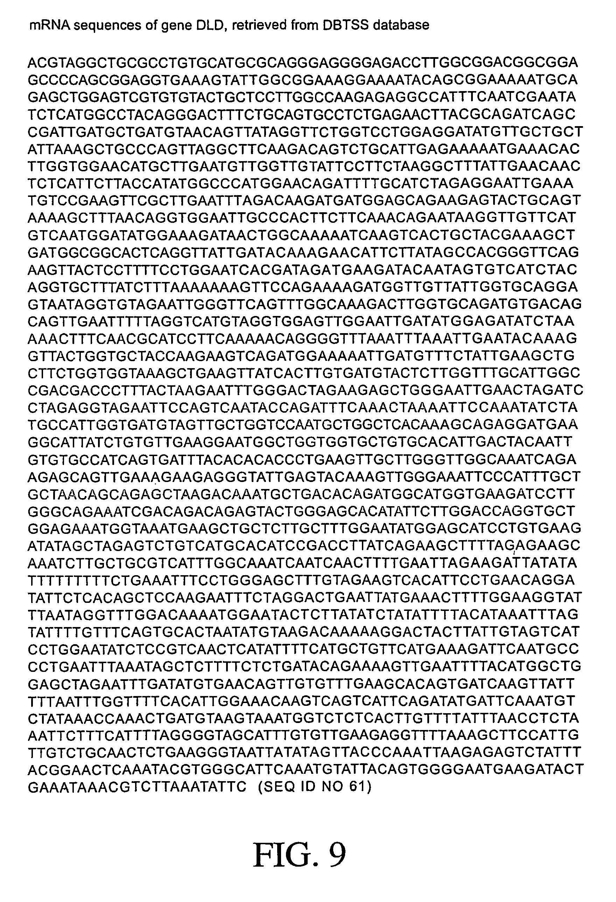

As illustrated in Figure 2, three different versions of full-length mRNA sequences can be retrieved from NCBI database (refseq), MGC database (MGC), Japan DBTSS database (DBTSS), and the like. The completely sequences for gene DLD retrieved from refseq database is listed in Figure 7 (SEQ ID NO 59), and the one retrieved from MGC is listed in Figure 8 (SEQ ID NO 60), and the one retrieved from DBTSS is listed in Figure 9 (SEQ ID NO 61).

### 2. The retrieved genes are mapped to a recently updated human genome.

A self-developed script is used to fetch the above retrieved sequence to UCSC genome browser database to map their genomic position. The retrieved different version of gene DLD are mapped to the recently updated human genome using a tool provided by at least one of public available UCSC genome browser databases.

### 3. The position of the TSS is mapped.

The mapped positions are retrieved using self-developed script from the above referenced UCSC genome browser database. The summary result of mapping is listed in table 1. For example, the full length gene DLD sequence from NCBI refseq database was mapped to the human genome working draft (released June 2002 by UCSC genome browser) at the chromosome 7 sense strand or positive strand, starting at the chromosome position of 106015510, ending at the chromosome position of 106044308.

**Table 1:**

| **name** | **chromosome** | **strand** | **start** | **end** |
|---|---|---|---|---|
| DLD from refseq | 7 | + | 106015510 | 106044308 |
| DLD from MGC | 7 | + | 106015541 | 106044089 |
| DLD from DBTSS | 7 | + | 106015488 | 106044308 |

### 4. The TSS is mapped by making the most 5-prime TSS of each gene after comparing all available TSS'S for the gene.

Referring again to Figure 2, this mapping is facilitated by using self-generated script. For gene DLD, since it is located on "+" strand of chromosome 7. The start position 106015488 is taken as the most 5' position for TSS of gene DLD.

### 5. A genomic sequence of a regulatory region for each retrieved gene with the most 5'TSS is retrieved from the most updated human genome.

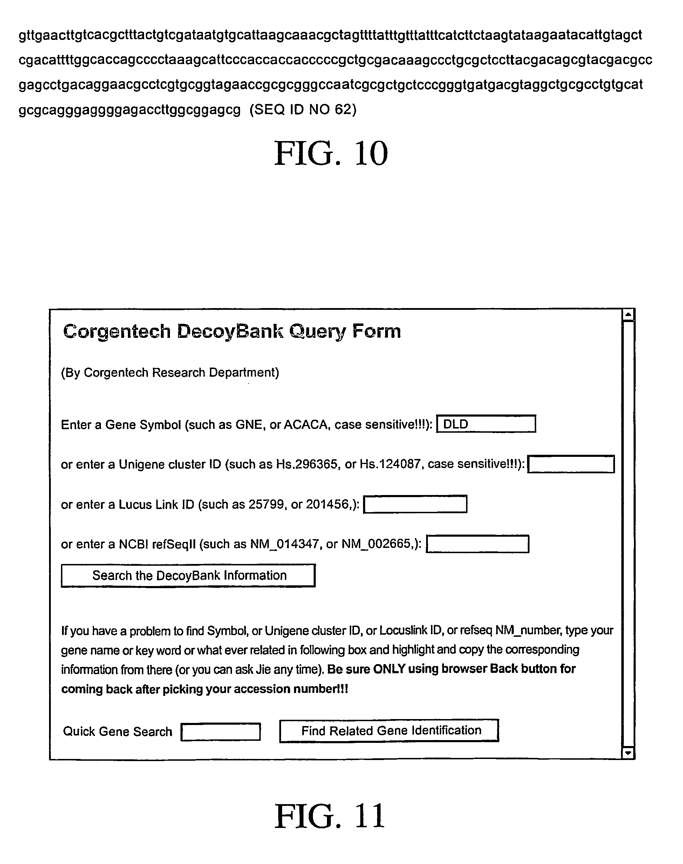

The 5' regulatory region is the sequences upstream of the TSS and downstream of the TSS. More specifically, for gene DLD, the regulatory region or core promoter region is the sequence includes 200-300 bases upstream and the sequence about 50-100 bases downstream of the TSS. Therefore, the corresponding sequences relative to TSS of gene DLD are cut and stored with the use of self-developed scripts from at least one of the UCSC genome browser or NCBI genome database. The stored sequence for gene DLD is listed in Figure 10 (SEQ ID NO 62).

### 6. The stored sequence for regulatory region of gene DLD is screened using a match program.

The MATCH program is the sequence-analyzing tool embedded inside the licensed TRANSFAC database. The analysis is done with the proper setting for both the scores of matrix similarity and scores of core similarity in order to reduce the false positive and false negative matching during screening. The result of the screening for the regulatory region of gene DLD is shown in table 2 where the positions of identified binding sites are listed.

**Table 2:**

| **position** | **strand** | **core score** | **matrix score** | **sequences** | **TF name** |
|---|---|---|---|---|---|
| 3 | (+) | 1 | 0.964 | tgaacttgTCACGctttactg (SEQ ID NO 4) | Pax-3 |
| 5 | (-) | 0.796 | 0.779 | aacttgtcacgCTTTActgtc (SEQ ID NO 5) | Pax-4 |
| 6 | (+) | 1 | 0.886 | acttgTCACGctttactgtcg (SEQ ID NO 6) | Pax-6 |
| 6 | (+) | 0.977 | 0.761 | acttgTCACGctttactgtcg (SEQ ID NO 7) | Pax-4 |
| 24 | (+) | 0.994 | 0.972 | tCGATAatg (SEQ ID NO 8) | Lmo2 complex |
| 25 | (-) | 0.951 | 0.963 | cgataatgtgCATTAagc (SEQ ID NO 9) | Cart-1 |
| 25 | (+) | 0.951 | 0.952 | cgaTAATGtgcattaagc (SEQ ID NO 10) | Cart-1 |
| 34 | (+) | 0.896 | 0.772 | gcaTTAAGcaaa (SEQ ID NO 11) | Cdc5 |
| 48 | (+) | 1 | 0.806 | ctagtTTTATttgt (SEQ ID NO 12) | Cdx-2 |
| 50 | (-) | 0.96 | 0.937 | agtttTATTTgtttattt (SEQ ID NO 13) | FOXJ2 |
| 50 | (+) | 1 | 0.952 | agtttTATTTgttta (SEQ ID NO 14) | HNF-3 beta |
| 51 | (+) | 1 | 0.938 | gttttATTTGttt (SEQ ID NO 15) | Xvent-1 |
| 52 | (+) | 0.948 | 0.926 | ttTTATTtgttt (SEQ ID NO 16) | FOXD3 |
| 52 | (+) | 0.981 | 0.98 | tttTATTTgttta (SEQ ID NO 17) | HFH-3 |
| 54 | (-) | 1 | 0.982 | ttattTGTTTatttcatc (SEQ ID NO 18) | FOXJ2 |
| 54 | (+) | 1 | 0.926 | aattTGTTTatttc (SEQ ID NO 19) | HNF-3 beta |
| 55 | (-) | 1 | 0.947 | tatttgTTTATttc (SEQ ID NO 20) | XFD-2 |
| 55 | (-) | 1 | 0.983 | tatttgTTTATttcat (SEQ ID NO 21) | Freac-7 |
| 55 | (-) | 1 | 0.996 | tattTGTTTat (SEQ ID NO 22) | FOXO4 |
| 55 | (+) | 0.972 | 0.97 | TATTTgtttat (SEQ ID NO 23) | HNF-3 alpha |
| 56 | (+) | 1 | 0.834 | atttgTTTATttca (SEQ ID NO 24) | Cdx-2 |
| 56 | (+) | 1 | 0.971 | attTGTITatttc (SEQ ID NO 25) | HFH-3 |
| 56 | (+) | 1 | 0.986 | attTGTTTatttc (SEQ ID NO 26) | HFH-8 |
| 56 | (+) | 1 | 0.963 | atttGTTTAttt (SEQ ID NO 27) | HFH-1 |
| 56 | (+) | 1 | 0.958 | atTTGTTtattt (SEQ ID NO 28) | FOXD3 |
| 59 | (+) | 1 | 0.919 | TGTTTatttca (SEQ ID NO 29) | HNF-3alpha |
| 60 | (-) | 0.85 | 0.878 | gtttatttcatCTTCTaa (SEQ ID NO 30) | IRF-7 |
| 72 | (+) | 1 | 0.964 | ttctAAGTAtaa (SEQ ID NO 31) | NKX3A |
| 72 | (+) | 0.824 | 0.873 | ttctaAGTATaagaatacattgta (SEQ ID NO 32) | STAT5A (homotetramer) |
| 123 | (-) | 1 | 0.962 | agcaTTCCCacca (SEQ ID NO 33) | lk-1 |
| 123 | (-) | 1 | 0.927 | agcaTTCCCacca (SEQ ID NO 34) | lk-3 |
| 147 | (-) | 0.813 | 0.869 | gCGACAaa (SEQ ID NO 35) | E2F |
| 154 | (-) | 0.789 | 0.755 | agccctgcgctCCTTAcgaca (SEQ ID NO 36) | Pax-4 |
| 202 | (-) | 0.96 | 0.925 | gcctCGTGCg (SEQ ID NO 37) | USF |
| 222 | (+) | 1 | 0.934 | gcgggCCAATcg (SEQ ID NO 38) | CCAATbox |
| 234 | (-) | 0.788 | 0.784 | cgctgctcccgGGTGAtgacg (SEQ ID NO 39) | Pax-4 |
| 237 | (-) | 0.964 | 0.902 | tgctcccgggTGATGacgtag (SEQ ID NO 40) | Muscle initiator sequence-20 |
| 244 | (+) | 0.91 | 0.839 | gggtGATGAcgtaggctgc (SEQ ID NO 41) | v-Maf |
| 246 | (+) | 1 | 0.991 | gtgaTGACGtag (SEQ ID NO 42) | CREB |
| 248 | (+) | 1 | 0.881 | gaTGACGtaggc (SEQ ID NO 43) | ATF4 |
| 248 | (+) | 1 | 0.954 | gaTGACGtaggc (SEQ ID NO 44) | CREB |
| 250 | (+) | 0.973 | 0.951 | tgacGTAGG (SEQ ID NO 45) | TFII-I |
| 250 | (+) | 1 | 0.971 | tGACGtag (SEQ ID NO 46) | CREB |
| 277 | (+) | 1 | 0.97 | aGGGAGgg (SEQ ID NO 47) | MAZ |
| 290 | (+) | 1 | 0.999 | cTTGGCgg (SEQ ID NO 48) | E2F-1 |
| 290 | (+) | 0.964 | 0.916 | ctTGGCGg (SEQ ID NO 49) | E2F-1 |
| 290 | (+) | 0.984 | 0.897 | ctTGGCGg (SEQ ID NO 50) | E2F |

### 7. A genomic or tissue specific frequency of each binding site is determined.

The frequency is the existence of specific TF binding sites in regulatory regions of all the genes or tissue specific genes. After analysis of the regulatory region of all the genes, the frequency or probability of existence of TF binding sites is easy established. Some of these frequencies information are listed for gene DLD in table 3:

**Table 3:**

| **TF name** | **left position** | **right position** | **distance (base) to TSS** | **genomic frequency** |
|---|---|---|---|---|
| Pax-3 | 106015239 | 106015259 | -249 | 0.426259226 |
| Pax-4 | 106015241 | 106015261 | -247 | 0.96109025 |
| Pax-6 | 106015242 | 106015262 | -246 | 0.112003108 |
| Pax-4 | 106015242 | 106015262 | -246 | 0.96109025 |
| Lmo2complex | 106015260 | 106015268 | -228 | 0.120419526 |
| Cart-1 | 106015261 | 106015278 | -227 | 0.020134663 |
| Cart-1 | 106015261 | 106015278 | -227 | 0.020134663 |
| Cdc5 | 106015270 | 106015281 | -218 | 0.360481678 |
| Cdx-2 | 106015284 | 106015297 | -204 | 0.259031464 |
| FOXJ2 | 106015286 | 106015303 | -202 | 0.167875178 |
| HNF-3beta | 106015286 | 106015300 | -202 | 0.23688981 |
| Xvent-1 | 106015287 | 106015299 | -201 | 0.678946005 |
| HFH-3 | 106015288 | 106015300 | -200 | 0.066942898 |
| FOXD3 | 106015288 | 106015299 | -200 | 0.653632008 |
| FOXJ2 | 106015290 | 106015307 | -198 | 0.167875178 |
| HNF-3beta | 106015290 | 106015304 | -198 | 0.23688981 |
| FOXO4 | 106015291 | 106015301 | -197 | 0.10785964 |
| XFD-2 | 106015291 | 106015304 | -197 | 0.033665674 |
| Freac-7 | 106015291 | 106015306 | -197 | 0.076718892 |
| HNF-3alpha | 106015291 | 106015301 | -197 | 0.312184384 |
| HFH-1 | 106015292 | 106015303 | -196 | 0.01657387 |
| HFH-3 | 106015292 | 106015304 | -196 | 0.066942898 |
| HFH-8 | 106015292 | 106015304 | -196 | 0.020652596 |
| FOXD3 | 106015292 | 106015303 | -196 | 0.653632008 |
| Cdx-2 | 106015292 | 106015305 | -196 | 0.259031464 |
| HNF-3alpha | 106015295 | 106015305 | -193 | 0.312184384 |
| IRF-7 | 106015296 | 106015313 | -192 | 0.206914411 |
| NKX3A | 106015308 | 106015319 | -180 | 0.02233588 |
| STAT5A(homotetramer) | 106015308 | 106015331 | -180 | 0.02926324 |
| lk-1 | 106015359 | 106015371 | -129 | 0.149682766 |
| lk-3 | 106015359 | 106015371 | -129 | 0.019875696 |
| E2F | 106015383 | 106015390 | -105 | 0.566230739 |
| Pax-4 | 106015390 | 106015410 | -98 | 0.96109025 |
| USF | 106015438 | 106015447 | -50 | 0.781561569 |
| CCAATbox | 106015458 | 106015469 | -30 | 0.288488929 |
| Pax-4 | 106015470 | 106015490 | -18 | 0.96109025 |
| Muscleinitiator sequence-20 | 106015473 | 106015493 | -15 | 0.29004273 |
| v-Maf | 106015480 | 106015498 | -8 | 0.233458501 |
| CREB | 106015482 | 106015493 | -6 | 0.308429367 |
| CREB | 106015484 | 106015495 | -4 | 0.308429367 |
| ATF4 | 106015484 | 106015495 | -4 | 0.142172731 |
| TFII-I | 106015486 | 106015494 | -2 | 0.949177781 |
| CREB | 106015486 | 106015493 | -2 | 0.308429367 |
| MAZ | 106015513 | 106015520 | 25 | 1.118477276 |
| E2F | 106015526 | 106015533 | 38 | 0.566230739 |
| E2F-1 | 106015526 | 106015533 | 38 | 0.901268937 |
| E2F-1 | 106015526 | 106015533 | 38 | 0.901268937 |

### 8. A conservation score for each binding site is created.

The conservation scores for whole genome comparison between human and mouse are retrieved from UCSC genome browser database. The conservation score is selected to cover regions where the TF binding sites are identified. The conservation scores for the TF binding sites identified in the regulatory region of gene DLD are listed in table 4.

**Table 4:**

| **TF name** | **core sequences** | **start position** | **end position** | **distance to TSS** | **Conservati on score** |
|---|---|---|---|---|---|
| Pax-3 | tgaacttgTCACGctttactg (SEQ ID NO 4) | 106015239 | 106015259 | -249 | 0.426 |
| Pax-4 | aacttgtcacgCTTTActgtc (SEQ ID NO 5) | 106015241 | 106015261 | -247 | 0.3552 |
| Pax-6 | acttgTCACGctttactgtcg (SEQ ID NO 6) | 106015242 | 106015262 | -246 | 0.3552 |
| Pax-4 | acttgTCACGctttactgtcg (SEQ ID NO 7) | 106015242 | 106015262 | -246 | 0.3552 |
| Lmo2complex | tCGATAatg (SEQ ID NO 8) | 106015260 | 106015268 | -228 | 0.06 |
| Cart-1 | cgaTAATGtgcattaagc (SEQ ID NO 10) | 106015261 | 106015278 | -227 | 0.06 |
| Cart-1 | cgataatgtgCATTAagc (SEQ ID NO 9) | 106015261 | 106015278 | -227 | 0.06 |
| Cdc5 | gcaTTAAGcaaa (SEQ ID NO 11) | 106015270 | 106015281 | -218 | 0.064 |
| Cdx-2 | ctagtTTTATttgt (SEQ ID NO 12) | 106015284 | 106015297 | -204 | 0.11 |
| FOXJ2 | agtttTATTTgtttattt (SEQ ID NO 13) | 106015286 | 106015303 | -202 | 0.162 |
| HNF-3beta | agtttTATTTgttta (SEQ ID NO 14) | 106015286 | 106015300 | -202 | 0.162 |
| Xvent-1 | gttttATTTGttt (SEQ ID NO 15) | 106015287 | 106015299 | -201 | 0.1226666 67 |
| HFH-3 | tttTATTTgttta (SEQ ID NO 17) | 106015288 | 106015300 | -200 | 0.162 |
| FOXD3 | ttTTATTtgttt (SEQ ID NO 16) | 106015288 | 106015299 | -200 | 0.1226666 67 |
| FOXJ2 | ttattTGTTTatttcatc (SEQ ID NO 18) | 106015290 | 106015307 | -198 | 0.286 |
| HNF-3beta | ttattTGTTTatttc (SEQ ID NO 19) | 1060T5290 | 106015304 | -198 | 0.192 |
| FOX04 | tattTGTTTat (SEQ ID NO 22) | 106015291 | 106015301 | -197 | 0.192 |
| XFD-2 | tatttgTTTATttc (SEQ ID NO 20) | 106015291 | 106015304 | -197 | 0.192 |
| Freac-7 | tatttgTTTATttcat (SEQ ID NO 21) | 106575291 | 106015306 | -197 | 0.286 |
| HNF-3alpha | TATTTgtttat (SEQ ID NO 23) | 106015291 | 106015301 | -197 | 0.192 |
| HFH-1 | atttGTTTAttt (SEQ ID NO 27) | 106015292 | 106015303 | -196 | 0.192 |
| HFH-3 | attTGTTTatttc (SEQ ID NO 25) | 106015292 | 106015304 | -196 | 0.192 |
| HFH-8 | attTGTTTatttc (SEQ ID NO 26) | 106015292 | 106015304 | -196 | 0.192 |
| FOXD3 | atTTGTTtattt (SEQ ID NO 28) | 106015292 | 106015303 | -196 | 0.192 |
| Cdx-2 | atttgTTTATttca (SEQ ID NO 24) | 1060T5292 | 106015305 | -196 | 0.286 |
| HNF-3alpha | TGTTTatttca (SEQ ID NO 29) | 106015295 | 106015305 | -193 | 0.3573333 33 |
| IRF-7 | gtttatttcatCTTCTaa (SEQ ID NO 30) | 106015296 | 106015313 | -192 | 0.41 |
| NKX3A | ttctAAGTAtaa (SEQ ID NO 31) | 106015308 | 106015319 | -180 | 0.624 |
| STAT5A (homotetra mer) | | 106015308 | 106015331 | -180 | 0.5506666 67 |
| lk-1 | agcaTTCCCacca (SEQ ID NO 33) | 106015359 | 106015371 | -129 | 0.394 |
| lk-3 | agcaTTCCCacca (SEQ ID NO 34) | 106015359 | 106015371 | -129 | 0.394 |
| E2F | gCGACAaa (SEQ ID NO 35) | 106015383 | 106015390 | -105 | 0.6746666 67 |
| Pax-4 | | 106015390 | 106015410 | -98 | 1.2336 |
| USF | gcctCGTGCg (SEQ ID NO 37) | 106015438 | 106015447 | -50 | 0.888 |
| CCAAT-box | gcgggCCAATcg (SEQ ID NO 38) | 106015458 | 106015469 | -30 | 1.136 |
| Pax-4 | | 106015470 | 106015490 | -18 | 1.3408 |
| Muscle initiator sequence-20 | | 106015473 | 106015493 | -15 | 1.3408 |
| v-Maf | gggtGATGAcgtaggctgc (SEQ ID NO 41) | 106015480 | 106015498 | -8 | 1.356 |
| CREB | gtgaTGACGtag (SEQ ID NO 42) | 106015482 | 106015493 | -6 | 1.3786666 67 |
| CREB | gaTGACGtaggc (SEQ ID NO 44) | 106015484 | 106015495 | -4 | 1.356 |
| ATF4 | gaTGACGtaggc (SEQ ID NO 43) | 106015484 | 106015495 | -4 | 1.356 |
| TFII-I | tgacGTAGG (SEQ ID NO 45) | 106015486 | 106015494 | -2 | 1.544 |
| CREB | TGACGtag (SEQ ID NO 46) | 106015486 | 106015493 | -2 | 1.544 |
| MAZ | aGGGAGgg (SEQ ID NO 47) | 106015513 | 106015520 | 25 | 0.7146666 67 |
| E2F | ctTGGCGg (SEQ ID NO 50) | 106015526 | 106015533 | 38 | 0.532 |
| E2F-1 | ctTGGCGg (SEQ ID NO 49) | 106015526 | 106015533 | 38 | 0.532 |
| E2F-1 | cTTGGCgg (SEQ ID NO 48) | 106015526 | 106015533 | 38 | 0.532 |

### 9. A determination is made of the clustering of the binding sites and their positions.

The adjacent or overlapped binding sites are clustered by using self-generated script and the corresponding position and TF are listed in the table 5 for the gene DLD.

**Table 5:**

| Cluster ID | Core sequences | left position | right position | Transcription Factor(s) |
|---|---|---|---|---|
| | | | | Cdc5;Cart-1;Cart-1;Lmo2compIex ;Pax- |
| 1 | | 106015239 | 106015281 | 4;Pax-6;Pax-4;Pax-3; |
| | | | | STATSA(homotetramer);N KX3A;IRF- |
| | | | | 7;HNF-3alpha;Cdx;-2;HFH-3;HFH-8;HFH- |
| | | | | 1;FOXD3;Freac-7;XFD-2;HNF- |
| | | | | 3alpha;FOX04;FOXJ2;HN F-3beta;HFH- |
| | | | | 3;FOXD3;Xvent-1;FOXJ2;HNF-3beta;Cdx- |
| 2 | | 106015284 | 106015331 | 2; |
| 3 | agcattcccacca (SEQ ID NO 53) | 106015359 | 106015371 | lk-3;lk-1; |
| 4 | | 106015383 | 106015410 | Pax-4;E2F; |
| 5 | gcctcgtgcg (SEQ ID NO 55) | 106015438 | 106015447 | USF; |
| | | | | TFII-I;CREB;CREB;ATF4;CRE B;v- |
| | | | | Maf;Muscleinitiator sequence-20 ;Pax- |
| 6 | | 106015458 | 106015498 | 4;CCAATbox; |
| 7 | agggaggg (SEQ ID NO 57) | 106015513 | 105015520 | MAZ; |
| 8 | cttggcgg (SEQ ID NO 58) | 106015526 | 106015533 | E2F;E2F-1;E2F-1; |

### 10. Binding profiles are collected in the database.

All the binding profiles listed above are been collected in the database. The example list of the entry for gene DLD is shown in Table 6.

**Table 6:**

| **TF name** | **core score** | **matrix score** | **core sequences** | **left position** | **right position** | **distance (base) to TSS** | **genomic frequency** | **conservation score** |
|---|---|---|---|---|---|---|---|---|
| Pax-3 | 1 | 0.964 | tgaacttaTCACGctttactg (SEQ ID NO 63) | 106015239 | 106015259 | -249 | 0.426259226 | 0.426 |
| Pax-4 | 0.796 | 0.779 | aacttgtcacgCTTTActgtc (SEQ ID NO 5) | 106015241 | 106015261 | -247 | 0.96109025 | 0.3552 |
| Pax-6 | 1 | 0.886 | acttgTCACGctttactgtcg (SEQ ID NO 6) | 106015242 | 106015262 | -246 | 0.112003108 | 0.3552 |
| Pax-4 | 0.977 | 0.761 | acttgTCACGctttactgtcg (SEQ ID NO 7) | 106015242 | 106015262 | -246 | 0.96109025 | 0.3552 |
| Lmo2compl ex | 0.994 | 0.972 | tCGATAatg (SEQ ID NO 8) | 106015260 | 106015268 | -228 | 0.120419526 | 0.06 |
| Cart-1 | 0.951 | 0.952 | caaTAATGtgcattaagc (SEQ ID NO 64) | 106015261 | 106015278 | -227 | 0.020134663 | 0.06 |
| Cart-1 | 0.951 | 0.963 | cgataatgtCATTAagc (SEQ ID NO 9) | 106015261 | 106015278 | -227 | 0.020134663 | 0.06 |
| Cdc5 | 0.896 | 0.772 | gcaTTAAGcaaa (SEQ ID NO 11) | 106015270 | 106015281 | -218 | 0.360481678 | 0.064 |
| Cdx-2 | 1 | 0.806 | ctagtTTTATttgt (SEQ ID NO 12) | 106015284 | 106015297 | -204 | 0.259031464 | 0.11 |
| FOXJ2 | 0.96 | 0.937 | agtttTATTTgtttattt (SEQ ID NO 13) | 106015286 | 106015303 | -202 | 0.167875178 | 0.162 |
| HNF-3beta | 1 | 0.952 | agtttTATTTgttta (SEQ ID NO 14) | 106015286 | 106015300 | -202 | 0.23688981 | 0.162 |
| Xvent-1 | 1 | 0.938 | gttttATTTGttt (SEQ ID NO 15) | 106015287 | 106015299 | -201 | 0.678946005 | 0.122666667 |
| HFH-3 | 0.981 | 0.98 | tttTATTTgttta (SEQ ID NO 17) | 106015288 | 106015300 | -200 | 0.066942898 | 0.162 |
| FOXD3 | 0.948 | 0.926 | ttTTATTtgttt (SEQ ID NO 16) | 106015288 | 106015299 | -200 | 0.653632008 | 0.122666667 |
| FOXJ2 | 1 | 0.982 | ttattTGTTTatttcatc (SEQ ID NO 18) | 106015290 | 106015307 | -198 | 0.167875178 | 0.286 |
| HNF-3beta | 1 | 0.926 | ttattTGTTTatttc (SEQ ID NO 19) | 106015290 | 106015304 | -198 | 0.23688981 | 0.192 |
| FOX04 | 1 | 0.996 | tattTGTTTat (SEQ ID NO 22) | 106015291 | 106015301 | -197 | 0.10785964 | 0.192 |
| XFD-2 | 1 | 0.947 | tatttgTTTATttc (SEQ ID NO 20) | 106015291 | 106015304 | -197 | 0.033665674 | 0.192 |
| Freac-7 | 1 | 0.983 | tatttgTTTATttcat (SEQ ID NO 21) | 106015291 | 106015306 | -197 | 0.076718892 | 0.286 |
| HNF-3alpha | 0.972 | 0.97 | TATTTgtttat (SEQ ID NO 23) | 106015291 | 106015301 | -197 | 0.312184384 | 0.192 |
| HFH-1 | 1 | 0.963 | atttGTTTAttt (SEQ ID NO 27) | 106015292 | 106015303 | -196 | 0.01657387 | 0.192 |
| HFH-3 | 1 | 0.971 | attTGTTTatttc (SEQ ID NO 25) | 106015292 | 106015304 | -196 | 0.066942898 | 0.192 |
| HFH-8 | 1 | 0.986 | attTGTTTatttc (SEQ ID NO 26) | 106015292 | 106015304 | -196 | 0.020652596 | 0.192 |
| FOXD3 | 1 | 0.958 | atTTGTTtattt (SEQ ID NO 28) | 106015292 | 106015303 | -196 | 0.653632008 | 0.192 |
| Cdx-2 | 1 | 0.834 | atttgTTTATttca (SEQ ID NO 24) | 106015292 | 106015305 | -196 | 0.259031464 | 0.286 |
| HNF-3alpha | 1 | 0.919 | TGTTTatttca (SEQ ID NO 29) | 106015295 | 106015305 | -193 | 0.312184384 | 0.357333333 |
| IRF-7 | 0.85 | 0.878 | gtttatttcatCTTCTaa (SEQ ID NO 30) | 106015296 | 106015313 | -192 | 0.206914411 | 0.41 |
| NKX3A | 1 | 0.964 | ttctAAGTAtaa (SEQ ID NO 31) | 106015308 | 106015319 | -180 | 0.02233588 | 0.624 |
| STAT5A(ho motetramer) | 0.824 | 0.873 | ttctaAGTATaagaatacattgta (SEQ ID NO 32) | 106015308 | 106015331 | -180 | 0.02926324 | 0.550666667 |
| lk-1 | 1 | 0.962 | agcaTTCCCacca (SEQ ID NO 33) | 106015359 | 106015371 | -129 | 0.149682766 | 0.394 |
| lk-3 | 1 | 0.927 | agcaTTCCacca (SEQ ID NO 65) | 106015359 | 106015371 | -129 | 0.019875696 | 0.394 |
| E2F | 0.813 | 0.869 | gCGACAaa (SEQ ID NO 35) | 106015383 | 106015390 | -105 | 0.566230739 | 0.674666667 |
| Pax-4 | 0.789 | 0.755 | agccctgcgctCCTTAcgaca (SEQ ID NO 36) | 106015390 | 106015410 | -98 | 0.96109025 | 1.2336 |
| USF | 0.96 | 0.925 | gcctCGTGCg (SEQ ID NO 37) | 106015438 | 106015447 | -50 | 0.781561569 | 0.888 |
| CCAATbox | 1 | 0.934 | gcgggCCAATcg (SEQ ID NO 38) | 106015458 | 106015469 | -30 | 0.288488929 | 1.136 |
| Pax-4 | 0.788 | 0.784 | cgctgctcccgGGTGAtgacg (SEQ ID NO 39) | 106015470 | 106015490 | -18 | 0.96109025 | 1.3408 |
| Muscle initiator sequence-20 | 0.964 | 0.902 | tgctcccgggTGATGacgtag (SEQ ID NO 40) | 106015473 | 106015493 | -15 | 0.29004273 | 1.3408 |
| v-Maf | 0.91 | 0.839 | gggtGATGAcgtaggctgc (SEQ ID NO 41) | 106015480 | 106015498 | -8 | 0.233458501 | 1.356 |
| CREB | 1 | 0.991 | gtgaTGACGtag (SEQ ID NO 42) | 106015482 | 106015493 | -6 | 0.308429337 | 1.378666667 |
| CREB | 1 | 0.954 | gaTGACGtaggc (SEQ ID NO 44) | 106015484 | 106015495 | -4 | 0.308429367 | 1.356 |
| ATF4 | 1 | 0.881 | gaTGACGtaggc (SEQ ID NO 43) | 106015484 | 106015495 | -4 | 0.142172731 | 1.356 |
| TFII-1 | 0.973 | 0.951 | tgacGTAGG (SEQ ID NO 45) | 106015486 | 106015494 | -2 | 0.949177781 | 1.544 |
| CREB | 1 | 0.971 | TGACGtag (SEQ ID NO 46) | 106015486 | 106015493 | -2 | 0.308429367 | 1.544 |
| MAZ | 1 | 0.97 | aGGGAGgg (SEQ ID NO 47) | 106015513 | 106015520 | 25 | 1.118477276 | 0.714666667 |
| E2F | 0.984 | 0.897 | ctTGGCGg (SEQ ID NO 50) | 106015526 | 106015533 | 38 | 0.566230739 | 0.532 |
| E2F-1 | 0.964 | 0.916 | ctTGGCGg (SEQ ID NO 49) | 106015526 | 106015533 | 38 | 0.901268937 | 0.532 |
| E2F-1 | 1 | 0.999 | cTTGGCgg (SEQ ID NO 48) | 106015526 | 106015533 | 38 | 0.901268937 | 0.532 |

### 11. The database is searchable by gene identifiers.

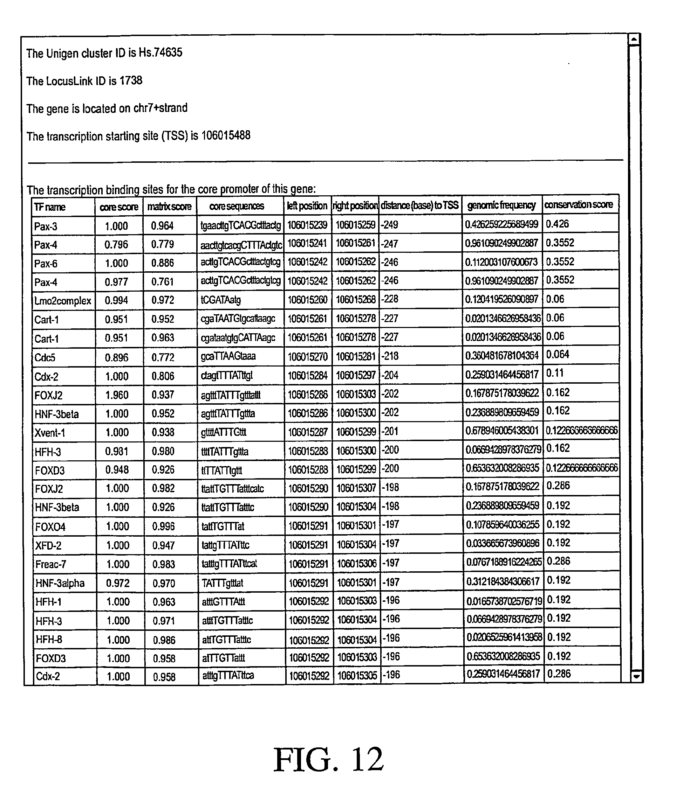

Figure 11 illustrates a screen shot of a query form that can be used with the database. Figure 12 illustrates a screen shot of a database query result

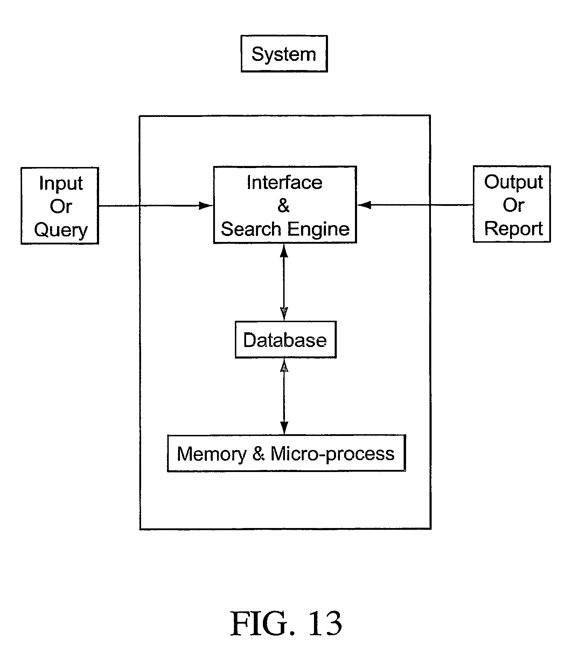

As illustrated in Figure 13, the present application also describes a computer implemented system for displaying the profiled regulatory factor binding sites. The system includes the database, a user interface that includes one or more selectable user inputs, an input device operable by a user, and a display for displaying at least one output in response to the profiled identified binding sites.

Examples of outputs include but are not limited to, gene name, identifier, identified TF binding site, TF names, genomic positions, length, distance, conservation score, binding scores, frequencies information, and binding sites sequences. Examples of inputs includes, the gene identifiers, such as gene symbols, unigene cluster ID, or locuslink ID, and the like.

The system also includes a memory, a microprocessor, data files, scripts, supporting available software, including but not limited to MS windows, red hat linux, Apache HTTP sever, Perl compiler program, and the like.

The foregoing description of a preferred embodiment of the invention has been presented for purposes of illustration and description. It is not intended to be exhaustive or to limit the invention to the precise forms disclosed. Obviously, many modifications and variations will be apparent to practitioners skilled in this art. It is intended that the scope of the invention be defined by the following claims.

## Claims

1. A method for profiling regulatory factor binding sites, comprising
locating and retrieving a complete and most 5' full-length gene for mapping the regulatory regions of said gene to a human genome;
retrieving genomic sequences from the human genome for the gene regulatory region comprising the sequence about 200-300 bases upstream of the transcriptional start site (TSS) and the sequence about 50-100 bases downstream of the TSS;
screening DNA sequence information of the retrieved gene regulatory region for the purpose of identifying putative regulatory factor binding sites;
creating a conservation score for each putative regulatory factor binding site;
profiling the putative regulatory factor binding sites of the gene;
collecting profiles including transcription factor binding site sequence information and the conservation score in a database; and
computationally generating all the possible transcriptional decoys for profiled regulatory factor binding sites.

2. The method of claim 1, wherein the retrieved genes are mapped to the human genome using a publicly available UCSC genome database browser.

3. The method of claim 1, wherein the TSS is mapped by taking the most 5' TSS of each gene after comparing all available TSSs for the gene.

4. The method of claim 1, wherein a genomic sequence of a regulatory region for each retrieved gene with the most 5'TSS is retrieved from a human genome.

5. The method of claim 4, further comprising:
cutting and storing the corresponding sequences relative to TSS.

6. The method of claim 1, wherein the DNA sequence information is screened using a MATCH program or the similar Position Weighted Matrix Programs for motif searching.

7. The method of claim 1, wherein the DNA sequence information screening includes selecting the TF matrix, scores of matrix similarity and scores of core similarity.

8. The method of claim 1, wherein cut-off is applied to reduce the false positive and false negative matching during screening.

9. The method of claim 1, further comprising:
determining at least one of a genomic or tissue-specific frequency of each binding site.

10. The method of claim 1, wherein the frequency is the existence of specific TF binding sites in regulatory regions of all the genes.

11. The method of claim 1, wherein the frequency is the existence of specific TF binding sites in regulatory regions of tissue specific genes.

12. The method of claim 7, further comprising:
creating a conservation score for each binding site.

13. The method of claim 12, wherein the conservation scores are selected to cover regions where the TF binding sites are identified.

14. The method of claim 8, further comprising:
determining a position of each binding site.

15. The method of claim 14, wherein the position is based on a human genome working draft.

16. The method of claim 15, wherein the position is a converted position in a human genome working draft.

17. The method of claim 14, wherein the genome position of a start and end is determined.

18. The method of claim 14, further comprising:
determining a distance of each binding site to a TSS.

19. The method of claim 18, wherein the distance is relative to a number of bases between a binding site and the TSS.

20. The method of claim 18, further comprising:
determining a length of each binding site.

21. The method of claim 20, further comprising:
determining sequence information about regions adjacent to the binding site.

22. The method of claim 21, further comprising:
determining co-existence information of other binding sites.

23. The method of claim 22, further comprising:
determining cluster of the binding sites and their positions.

24. The method of any one of the preceding claims, wherein the method further comprises experimentally screening said transcriptional decoys using high-throughput methods for binding efficiency optimization.

25. The method of claim 1, wherein the database includes TF binding profiles for the regulatory region of each gene.

26. The method of claim 1, wherein the database is searchable by gene identifiers.

27. The method of claim 1, wherein the gene identifiers are selected from the NCBI database.

28. The method of claim 27, wherein the NCBI database includes at least one of Unigene Cluster ID, LocusLink ID and international approved gene symbols.

29. The method of claim 26, wherein the database includes genomic frequencies information for TF.

30. The method 29 wherein the database includes genomic frequencies for vertebrate TF.

31. The method of claim 29 or claim 30, wherein the database can be sorted by at least one of TF name and TF frequencies.

32. The method of claim 31, wherein the TF frequencies include genome frequencies and tissue specific frequencies.

33. A method comprising profiling regulatory factor binding sites according to the method of claim 1, and further comprising:
retrieving information from the database for biomedical research;
retrieving information from the database for pre-clinical development;
retrieving information from the database for drug screening applications;
retrieving information from the database for target discovering and target validation;
retrieving information from the database for profiling of a regulatory region; or
retrieving information from the database for building the genome or tissue wide connections between regulatory profilings of different genes.

## Patentansprüche

1. Verfahren zur Profilerstellung von Regulationsfaktor-Bindungsstellen, umfassend
das Lokalisieren und Erhalten eines vollständigen und am meisten 5' gelegenen Gens voller Länge zur Kartierung der Regulationsregionen des Gens an ein menschliches Genom;
das Erhalten genomischer Sequenzen aus dem menschlichen Genom für die Regulationsregion des Gens, umfassend die Sequenz etwa 200-300 Basen stromauf der Transkriptionsstartstelle (TSS) und die Sequenz etwa 50-100 Basen stromab der TSS;
das Screenen von DNA-Sequenzinformationen der erhaltenen Gen-Regulationsregion zum Zweck der Identifikation mutmaßlicher Regulationsfaktor-Bindungsstellen;
das Erstellen eines Konservierungs-Scores für jede mutmaßliche Regulationsfaktor-Bindungsstelle;
das Erstellen eines Profils der mutmaßlichen Regulationsfaktor-Bindungsstellen des Gens;
das Sammeln von Profilen, umfassend Transkriptionsfaktor-Bindungsstellen-Sequenzinformationen sowie den Konservierungs-Score, in einer Datenbank; und
das computerunterstützte Erzeugen aller möglichen Transkriptions-Köder für Regulationsfaktor-Bindungsstellen, für die ein Profil erstellt wurde.

2. Verfahren nach Anspruch 1, worin die erhaltenen Gene unter Verwendung eines öffentlich erhältlichen UCSC-Genom-Datenbank-Browsers an das menschliche Genom kartiert werden.

3. Verfahren nach Anspruch 1, worin die TSS nach dem Vergleichen aller erhältlichen TSSs für das Gen durch das Heranziehen der am meisten 5' gelegenen TSS eines jeden Gens kartiert wird.

4. Verfahren nach Anspruch 1, worin eine genomische Sequenz einer Regulationsregion für jedes erhaltene Gen mit der am meisten 5' gelegenen TSS aus einem menschlichen Genom erhalten wird.

5. Verfahren nach Anspruch 4, weiters umfassend:
das Schneiden und Lagern der entsprechenden Sequenzen relativ zu TSS.

6. Verfahren nach Anspruch 1, worin die DNA-Sequenz-Information unter Verwendung eines MATCH-Programms oder des ähnlichen "Position-Weighted-Matrix"-Programms auf Motivsuche gescreent wird.

7. Verfahren nach Anspruch 1, worin das DNA-Sequenz-Informationsscreening das Auswählen der TF-Matrix, von Scores der Matrix-Ähnlichkeit und Scores der Kern-Ähnlichkeit umfasst.

8. Verfahren nach Anspruch 1, worin der Cut-off angewendet wird, um während des Screenings die falsch positive und falsch negative Übereinstimmung zu reduzieren.

9. Verfahren nach Anspruch 1, weiters umfassend:
das Bestimmen von zumindest einer genomischen oder gewebespezifischen Häufigkeit jeder Bindungsstelle.

10. Verfahren nach Anspruch 1, worin die Häufigkeit die Existenz spezifischer TF-Bindungsstellen in Regulationsregionen aller Gene darstellt.

11. Verfahren nach Anspruch 1, worin die Häufigkeit die Existenz spezifischer TF-Bindungsstellen in Regulationsregionen gewebespezifischer Gene darstellt.

12. Verfahren nach Anspruch 7, weiters umfassend:
das Erstellen eines Konservierungs-Scores für jede Bindungsstelle.

13. Verfahren nach Anspruch 12, worin die Konservierungs-Scores ausgewählt werden, um Regionen abzudecken, in denen die TF-Bindungsstellen identifiziert werden.

14. Verfahren nach Anspruch 8, weiters umfassend:
das Bestimmen einer Position jeder Bindungsstelle.

15. Verfahren nach Anspruch 14, worin die Position auf einem Arbeitsentwurf eines menschlichen Genoms basiert.

16. Verfahren nach Anspruch 15, worin die Position eine umgewandelte Position in einem Arbeitsentwurf eines menschlichen Genoms ist.

17. Verfahren nach Anspruch 14, worin die Genomposition eines Beginns und eines Endes bestimmt wird.

18. Verfahren nach Anspruch 14, weiters umfassend:
das Bestimmen eines Abstands jeder Bindungsstelle zu einer TSS.

19. Verfahren nach Anspruch 18, worin die Distanz relativ zu einer Anzahl an Basen zwischen einer Bindungsstelle und der TSS ist.

20. Verfahren nach Anspruch 18, weiters umfassend:
das Bestimmen einer Länge jeder Bindungsstelle.

21. Verfahren nach Anspruch 20, weiters umfassend:
das Bestimmen von Sequenzinformationen bezüglich Regionen, die an die Bindungsstelle angrenzen.

22. Verfahren nach Anspruch 21, weiters umfassend:
das Bestimmen von Co-Existenz-Informationen anderer Bindungsstellen.

23. Verfahren nach Anspruch 22, weiters umfassend:
das Bestimmen von Bindungsstellen-Clustern und ihrer Positionen.

24. Verfahren nach einem der vorangegangenen Ansprüche, worin das Verfahren wieters das experimentelle Screening der Transkriptions-Köder unter Verwendung von Verfahren mit hohem Durchsatz zur Optimierung der Bindungswirksamkeit umfasst.

25. Verfahren nach Anspruch 1, worin die Datenbank TF-Bindungsprofile für die Regulationsregion jedes Gens umfasst.

26. Verfahren nach Anspruch 1, worin die Datenbank mittels Gen-Identifikatoren durchsuchbar ist.

27. Verfahren nach Anspruch 1, worin die Gen-Identifikatoren aus der NCBI-Datenbank ausgewählt werden.

28. Verfahren nach Anspruch 27, worin die NCBI-Datenbank zumindest eines der Unigene-Cluster-ID-, LocusLink-ID- und der international anerkannten Gen-Symbole umfasst.

29. Verfahren nach Anspruch 26, worin die Datenbank Informationen über genomische Häufigkeiten von TF umfasst.

30. Verfahren 29, worin die Datenbank genomische Häufigkeiten von Wirbeltier-TF umfasst.

31. Verfahren nach Anspruch 29 oder Anspruch 30, worin die Datenbank durch zumindest eine(n) der TF-Namen und TF-Häufigkeiten sortiert werden kann.

32. Verfahren nach Anspruch 31, worin die TF-Häufigkeiten Genom-Häufigkeiten und gewebespezifische Häufigkeiten umfassen.

33. Verfahren, umfassend das Erstellen eines Profils von Regulationsfaktor-Bindungsstellen nach dem Verfahren nach Anspruch 1, weiters umfassend:
das Erhalten von Informationen aus der Datenbank für biomedizinische Forschung;
das Erhalten von Informationen aus der Datenbank für präklinische Entwicklung;
das Erhalten von Informationen aus der Datenbank für Arzneimittelscreening-Anwendungen;
das Erhalten von Informationen aus der Datenbank für Target-Entdeckung und Target-Validierung;
das Erhalten von Informationen aus der Datenbank für die Profilerstellung einer Regulationsregion; oder
das Erhalten von Informationen aus der Datenbank für den Aufbau der genom- oder gewebeweiten Verbindungen zwischen Regulationsprofilen unterschiedlicher Gene.

## Revendications

1. Procédé de profilage de sites de liaison de facteur régulateur, comprenant
la localisation et la récupération d'un gène de longueur totale le plus en 5' et complet pour mapper les régions régulatrices dudit gène sur un génome humain;
la récupération de séquences génomiques du génome humain pour la région régulatrice de gène comprenant la séquence d'environ 200 à 300 bases en amont du site de démarrage de transcription (TSS) et la séquence d'environ 50 à 100 bases en aval du TSS;
le criblage d'informations de séquence d'ADN de la région régulatrice de gène récupéré dans le but d'identifier les sites de liaison de facteur régulateur putatif;
la création d'une note de conservation pour chaque site de liaison de facteur régulateur putatif;
le profilage des sites de liaison de facteur régulateur putatif du gène;
la collecte de profils comprenant des informations de séquence de site de liaison de facteur de transcription et la note de conservation dans une base de données; et
la génération par ordinateur de tous les leurres de transcription possibles pour les sites de liaison de facteur régulateur profilé.

2. Procédé selon la revendication 1, dans lequel les gènes récupérés sont mappés sur le génome humain en utilisant un navigateur de base de données de génome UCSC disponible au public.

3. Procédé selon la revendication 1, dans lequel le TSS est mappé en prenant le TSS le plus en 5' de chaque gène après avoir comparé tous les TSS disponibles pour le gène.

4. Procédé selon la revendication 1, dans lequel une séquence génomique d'une région régulatrice pour chaque gène récupéré avec le TSS le plus en 5' est récupérée d'un génome humain.

5. Procédé selon la revendication 4, comprenant en outre:
la découpe et le stockage des séquences correspondantes par rapport au TSS.

6. Procédé selon la revendication 1, dans lequel les informations de séquence d'ADN sont criblées en utilisant un programme MATCH ou les programmes de matrice à position pondérée similaires pour la recherche de motif.

7. Procédé selon la revendication 1, dans lequel le criblage d'informations de séquence d'ADN comprend la sélection de la matrice TF, des notes de similarité de matrice et des notes de similarité essentielle.

8. Procédé selon la revendication 1, dans lequel une coupure est appliquée pour réduire la correspondance positive fausse et négative fausse pendant le criblage.

9. Procédé selon la revendication 1, comprenant en outre:
la détermination d'au moins l'une d'une fréquence génomique ou spécifique à un tissu de chaque site de liaison.

10. Procédé selon la revendication 1, dans lequel la fréquence est l'existence de sites de liaison TF spécifiques dans des régions régulatrices de tous les gènes.

11. Procédé selon la revendication 1, dans lequel la fréquence est l'existence de sites de liaison TF spécifiques dans des régions régulatrices de gènes spécifiques à un tissu.

12. Procédé selon la revendication 7, comprenant en outre:
la création d'une note de conservation pour chaque site de liaison.

13. Procédé selon la revendication 12, dans lequel les notes de conservation sont sélectionnées pour couvrir les régions où les sites de liaison TF sont identifiés.

14. Procédé selon la revendication 8, comprenant en outre:
la détermination d'une position de chaque site de liaison.

15. Procédé selon la revendication 14, dans lequel la position est basée sur une ébauche de travail de génome humain.

16. Procédé selon la revendication 15, dans lequel la position est une position convertie dans une ébauche de travail de génome humain.

17. Procédé selon la revendication 14, dans lequel la position de génome d'un début et d'une fin est déterminée.

18. Procédé selon la revendication 14, comprenant en outre:
la détermination d'une distance de chaque site de liaison à un TSS.

19. Procédé selon la revendication 18, dans lequel la distance est relative à un nombre de bases entre un site de liaison et le TSS.

20. Procédé selon la revendication 18, comprenant en outre:
la détermination d'une longueur de chaque site de liaison.

21. Procédé selon la revendication 20, comprenant en outre:
la détermination d'informations de séquence sur des régions adjacentes au site de liaison.

22. Procédé selon la revendication 21, comprenant en outre:
la détermination d'informations de co-existence d'autres sites de liaison.

23. Procédé selon la revendication 22, comprenant en outre:
la détermination d'un groupe des sites de liaison et leurs positions.

24. Procédé selon l'une quelconque des revendications précédentes, dans lequel le procédé comprend en outre le criblage expérimental desdits leurres de transcription en utilisant des procédés à rendement élevé pour l'optimisation d'efficacité de liaison.

25. Procédé selon la revendication 1, dans lequel la base de données comprend des profils de liaison TF pour la région régulatrice de chaque gène.

26. Procédé selon la revendication 1, dans lequel la base de données peut faire l'objet d'une recherche par des identifiants de gène.

27. Procédé selon la revendication 1, dans lequel les identifiants de gène sont sélectionnés dans la base de données NCBI.

28. Procédé selon la revendication 27, dans lequel la base de données NCBI comprend au moins l'un d'un ID de groupe unigène, d'un ID de liaison de locus et de symboles de gène approuvés internationaux.

29. Procédé selon la revendication 26, dans lequel la base de données comprend des informations de fréquence génomique pour TF.

30. Procédé selon la revendication 29, dans lequel la base de données comprend des fréquences génomiques pour TF de vertébrés.

31. Procédé selon la revendication 29 ou la revendication 30, dans lequel la base de données peut être triée par au moins l'un d'un nom TF et de fréquences TF.

32. Procédé selon la revendication 31, dans lequel les fréquences TF comprennent des fréquences de génome et des fréquences spécifiques à un tissu.

33. Procédé comprenant le profilage de sites de liaison de facteur régulateur selon le procédé de la revendication 1 et comprenant en outre:
la récupération d'informations de la base de données pour recherche biomédicale;
la récupération d'informations de la base de données pour développement pré-clinique;
la récupération d'informations de la base de données pour applications de criblage de médicament;
la récupération d'informations de la base de données pour découverte cible et validation cible;
la récupération d'informations de la base de données pour profilage d'une région régulatrice; ou
la récupération d'informations de la base de données pour créer les connexions de génome ou de tissu entre les profilages régulateurs de différents gènes.
